# EUROPEAN PATENT SPECIFICATION

(11) **EP 0 361 817 B1**
(45) Date of publication and mention of the grant of the patent: **28.08.2002**
(21) Application number: 89309705.5
(22) Date of filing: 25.09.1989
(51) Int. Cl.: C07D 219/04, G01N 33/533, G01N 33/82, G01N 33/74, G01N 33/88

(54) **Nucleophilic polysubstituted aryl acridinium esters, conjugates, production and uses thereof**
Nukleophile polysubstituierte Arylacridiniumester, deren Konjugate, sowie deren Herstellung und Verwendungen
Esters nucléophiles polysubstitués d'arylacridinium, conjugués, préparation et utilisation

(30) Priority: 26.09.1988 US 249620
(43) Date of publication of application: 04.04.1990
(60) Divisional application: 95200207.9
(73) Proprietor: Bayer Corporation, East Walpole, MA 02032 (US)
(72) Inventor: Chang, Steve Chin-Shen, Franklin, MA 02038 (US); Klukas, Carol K., Foxboro, MA 02035 (US); Law, Say-Jong, Westwood, MA 02090 (US); Vitkauskas, Christine Anne, Foxboro, MA 02035 (US)
(74) Representative: Froud, Clive

(56) References cited:
- EP-A- 0 082 636
- EP-A- 0 263 657
- CHEMICAL ABSTRACTS, vol. 97, no. 11, 13th September 1982, pages 111-112, abstract no. 85407p, Columbus, Ohio, US; A. PATEL et al.: "The preparation and properties of a chemiluminescent derivative of 173-estradiol", & SERONO SYMP. PUBL. RAVEN PRESS 1982, 1(LUMIN. ASSAYS: PERSPECT. ENDOCRINOL. CLIN. CHEM.), 181-9
- CLINICAL CHEMISTRY, vol. 29, no. 8, August 1983, pages 1474-1479, Washington, DC, US; I. WEEKS et al.: "Acridinium esters as high-specific-activity labels in immunoassay"
- CLINICAL CHEMISTRY, vol. 31, no. 10, October 1985, pages 1664-1668, Washington, DC, US; A.P. RICHARDSON et al.: "Chemiluminescence immunoassay of plasma progesterone, with progesterone-acridinium ester used as the labeled antigen"

## Description

This invention relates to nucleophilic polysubstituted aryl acridinium esters and to conjugates, production and uses thereof; more particularly, it relates to assays utilizing the nucleophilic polysubstituted aryl acridinium esters and conjugates thereof.

The use of acridinium esters as chemiluminescent labels in clinical assays is known. For example, European Patent No. 82636 describes the use of an aryl acridinium ester linked to an N-succinimidyl moiety as a chemiluminescent label in immunoassays. However, these compounds have now been found not to be stable in pH 7.4 buffer media and, therefore, are not ideal for use in commercial assays. U.S. Patent Nos. 4,745,181; 5,110,932 and 4,918,192 and related EP 263657.describe polysubstituted aryl acridinium esters which are useful in immunoassays and nucleic acid hybridization assays. The use of compounds in assays involving liposomes is also known. EP 353971 describes hydrophilic polysubstituted aryl acridinium esters and conjugates thereof useful in clinical assays, particularly those assays involving liposomes.

Weeks, et al (Clin. Chem. 29/8, 1474-1479, 1983) describe chemiluminescent acridinium esters which react spontaneously with proteins to yield stable, immunoactive derivatives of high specific activity. There is no teaching of further substitution on the phenoxy moiety or of the inclusion of nucleophilic groups as substituents and the effects on the luminescent characteristics of the compound having such substituents.

Richardson et al (Clin. Chem. 31/10, 1664-1668, 1985) and Miller et al (Ann. Clin. Biochem 25, 27-34, 1988) describe the use of (2-aminoethyl)-4-phenyl acridine-9-carboxylate in a chemiluminescent immunoassay for plasma progesterone.

However, the prior art acridinium esters often cannot effectively form conjugates with certain analytes. These analytes may lack nucleophilic groups or may contain carboxylic groups which are not readily amenable to modifications. In some cases, the nucleophilic group of the analyte cannot be acylated with an active-group containing acridinium ester because of the resulting deleterious effect on the immunoactivity of the analyte.

The acridine ester of Richardson et al and Miller et al cannot be converted to a useful acridinium ester without the concurrent loss of the nucleophilicity of the ester. The acridine ester must be conjugated first with the target analyte through a nucleophilic reaction, and then subsequently converted to an acridinium ester moiety. The general reaction conditions of the acridine ester-to-acridinium ester conversion are non-selective. As a result, susceptible groups on the target analytes are frequently affected, resulting in the loss of or reduction of the immunoactivity of the resulting conjugate.

Accordingly, it is an object of the present invention to provide certain advantageous nucleophilic polysubstituted aryl acridinium esters, which may contain an additional ionizable group.

It is a further object of the present invention to provide conjugates formed from the nucleophilic polysubstituted aryl acridinium esters.

It is another object of the present invention to provide assays utilizing the nucleophilic polysubstituted aryl acridinium esters and conjugates thereof.

The present invention provides an acridinium ester characterised in that it corresponds to the following general formula: 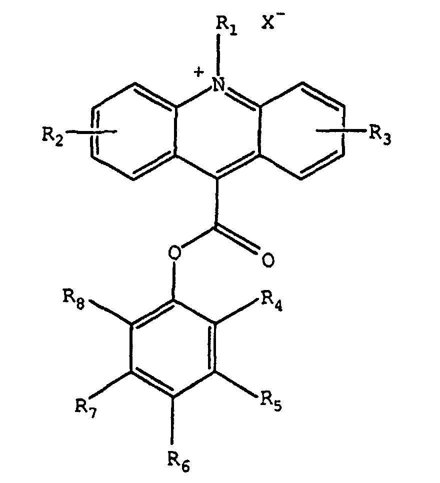 wherein
R₁ represents alkyl, alkenyl, alkynyl or aryl which contain up to 24 carbon atoms and which may contain up to 20 heteroatoms selected from nitrogen, oxygen, phosphorus and sulfur, or aralkyl which may contain one or more heteroatoms;
R₂, R₃, R₅ and R₇ independently represent hydrogen, amino, amido, acyl, alkoxyl, hydroxyl, -COON, halide, nitro, -CN, -SO₃H, 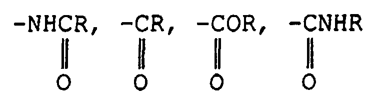 or -SCN; wherein R represents alkyl, alkenyl, alkynyl or aryl which contain up to 24 carbon atoms and which may contain up to 20 heteroatoms selected from nitrogen, oxygen, phosphorus and sulfur, or aralkyl which may contain one or more heteroatoms;
R₄ and R₈ independently represent alkyl, alkenyl, alkynyl, aralkyl or alkoxyl, which contain up to 8 carbon atoms;
R₆ represents Q-R-Nu or 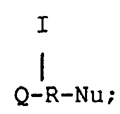 wherein Q represents -O-, -S-, -NH-, 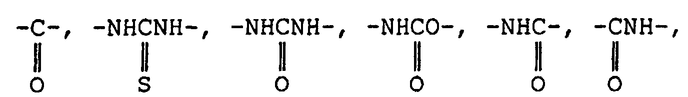 diazo or 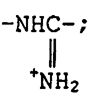 R is as defined above; I represents -SO₃H, -OSO₃H, -PO(OH)₂, -OPO(OH)₂ or -COOH; Nu represents the nucleophilic group amino; and X represents an anion; provided that, when Q represents -C(O)- and R represents -CH₃, I represents other than -SO₃H, and, when Q represents -C(O)- and R represents -C₂H₅, I represents other than -PO(OH)₂.

More particularly, it relates to such acridinium esters wherein R₁ represents alkyl, alkenyl, alkynyl or aryl, which contain up to 24 carbon atoms and which may contain up to 10 heteroatoms selected from nitrogen, oxygen, phosphorus and sulfur; R₂, R₃, R₅ and R₇ independently represent hydrogen, amino, -COOH, -CN, hydroxyl, C₁-C₄ alkoxyl, nitro, halide, -SO₃H or -SCN; R₄ and R₈ independently represent alkyl, alkenyl, alkynyl or alkoxyl, which contain up to 8 carbon atoms; X represents halide, CH₃SO₄-, FSO₃-, CF₃SO₃-, C₄F₉SO₃-, or 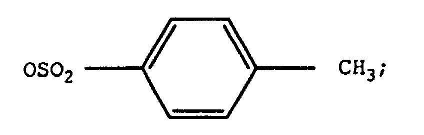 R contains up to 24 carbon atoms and may contain up to 10 heteroatoms selected from nitrogen, oxygen, phosphorus and sulfur; and Nu represents amino; preferably, R₁ represents C₁-C₁₀ alkyl; R₂, R₃, R₅ and R₇ independently represent hydrogen, C₁-C₄ alkoxyl, -CN, -SO₃H, nitro or amino; R₄ and R₈ independently represent C₁-C₄ alkyl; and X represents halide. More specifically, R₁, R₄ and R₈ represent methyl; R₂, R₃, R₅ and R₇ represent hydrogen; X represents bromide; and R₆ represents -CONH-CH₂CH₂-NH₂ or

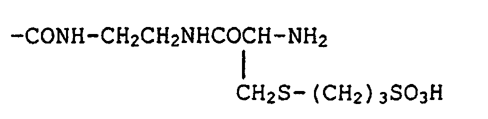

The present invention also provides a luminescent conjugate characterised in that it comprises such an acridinium ester covalently-bound to a biologically-active molecule, such as a protein, an antigen, a hapten, a nucleic acid, or a molecule comprising nucleic acids; preferably vitamin B₁₂ or a derivative thereof; or folate or a derivative thereof; or 17-beta-estradiol or a derivative thereof; or cortisol or a derivative thereof; or thromboxane B₂ or a prostaglandin analog.

The present invention further relates generally to the production of such esters and conjugates. Several specific instances may be exemplified. In one embodiment, the present invention provides a process for the production of a conjugate of folate or a derivative thereof and such an acridinium ester characterised in that it comprises;
(a) incubating the folate (derivative) with an amino-reactive compound to couple an acyl or alkyloxy carbonyl group to the C-2 amino group of the folate (derivative) so as to form a protected folate intermediate;
(b) activating at least one carboxylic group of the protected folate intermediate;
(c) incubating the activated protected folate intermediate with the acridinium ester so as to form an acridinium ester-protected folate intermediate conjugate; and
(d) incubating the acridinium ester-protected folate intermediate conjugate in an acidic medium to remove the amino-protecting group from the C-2 amino group, so as to form an acridinium ester-folate conjugate. In the case of vitamin B₁₂ or a derivative thereof, the process may comprise:
   (a) incubating the vitamin B₁₂ (derivative) in an acidic medium to deaminate the primary propanamide side chains of the Corrin ring of the vitamin B₁₂ (derivative) so as to produce a mixture of carboxylated vitamin B₁₂;
   (b) subjecting the mixture of carboxylated vitamin B₁₂ to HPLC to separate the tricarboxylic, the dicarboxylic, and the three monocarboxylic vitamin B₁₂ forms;
   (c) isolating one of the monocarboxylic vitamin B₁₂ forms;
   (d) activating the carboxylic group of the monocarboxylated vitamin B₁₂; and
   (e) incubating the activated monocarboxylic vitamin B₁₂ with the acridinium ester so as to produce an acridinium ester-vitamin B₁₂ conjugate. For 17-beta-estradiol or a derivative thereof, or cortisol or a derivative thereof, the production may comprise:
      (a) activating a functional group attached to the 17-beta-estradiol (derivative) or cortisol (derivative) so as to form an activated estradiol or cortisol intermediate; and
      (b) incubating the activated estradiol or cortisol intermediate with the acridinium ester so as to form an acridinium ester-17-beta-estradiol or -cortisol conjugate. The production of a conjugate of thromboxane B₂ or a prostaglandin may involve:
         (a) activating the terminal carboxylic group of the thromboxane B₂ or prostaglandin analog so as to form an activated intermediate of the thromboxane B₂ or the prostaglandin analog;
            and
         (b) incubating the activated intermediate with the acridinium ester so as to form an acridinium ester-thromboxane B₂ or acridinium ester-prostaglandin analog conjugate.

Furthermore, the present invention relates generally to the use of such acridinium esters and conjugates as chemiluminescent labels in assay systems. One method for the determination of vitamin B₁₂ or folate is characterised in that it comprises:
(a) incubating a sample fluid under conditions which will release vitamin B₁₂ or folate in the sample from any endogenous vitamin B₁₂ - or folate - binding proteins so as to produce a released sample fluid;
(b) incubating the released sample fluid with (i) a composite comprising a vitamin B₁₂ - or folate - binding protein immobilized on a solid support and (ii) an appropriate conjugate as above which may be produced by such a process so as to form a complexed composite;
(c) separating the complexed composite from any unbound conjugate;
(d) measuring the amount of acridinium ester label associated with the complexed composite; and
(e) determining the amount of vitamin B₁₂ or folate in the sample fluid from that measurement. More particularly, such a method may comprise:
   (a) incubating a sample fluid with (i) a base, preferably sodium hydroxide, and (ii) a sulfhydryl compound, preferably dithiothreitol, so as to produce a released sample fluid;
   (b) incubating the released sample fluid with (i) a composite comprising a vitamin B₁₂ - or folate - binding protein immobilized on a solid support and (ii) an appropriate conjugate as above, which may be produced by such a process so as to form a complexed composite;
   (c) separating the complexed composite from any unbound conjugate;
   (d) incubating the complexed composite with a thiol-reactive compound, preferably ethyl maleimide;
   (e) measuring the amount of acridinium ester label associated with the complexed composite; and
   (f) determining the amount of vitamin B₁₂ or folate in the sample fluid from that measurement. In the case of cortisol, such an assay may involve:
      (a) incubating a sample fluid with (i) a composite comprising an anti-cortisol antibody immobilized on a solid support and (ii) an appropriate conjugate as above, which may be produced by such a process so as to form a complexed composite;
      (b) separating the complexed composite from any unbound conjugate;
      (c) measuring the amount of acridinium ester label associated with the complexed composite; and
      (d) determining the amount of cortisol in the sample fluid from that measurement. For 17-beta-estradiol or thromboxane B₂, the method may comprise:
         (a) incubating a sample fluid with (i) an anti-17-beta-estradiol antibody or an anti-thromboxane B₂ antibody and (ii) an appropriate conjugate as above, which may be produced by such a process so as to form a reaction mixture;
         (b) incubating the reaction mixture with a composite comprising an antibody capable of binding to anti-17-beta-estradiol or anti-thromboxane B₂ antibody, which antibody is immobilized on a solid support, to form a complexed composite;
         (c) separating the complexed composite from the supernatant;
         (d) measuring the amount of acridinium ester label associated with the complexed composite; and
         (e) determining the amount of 17-beta-estradiol or thromboxane B₂ in the sample fluid from that measurement.

For the present purposes, Nu is amino, a nucleophilic chemical group which is electron-rich, has an unshared pair of electrons acting as a reactive site and seeks a positively-charged or electron-deficient site on another molecule.

As indicated, the present invention also relates to conjugates comprising the above-described acridinium esters covalently-bound to various compounds which can covalently bind to the Nu group of the acridinium ester. To form a useful conjugate, the compound must contain at least one functional group capable of binding to the Nu group of the acridinium ester. Preferably, the conjugates are formed from compounds having biological activity, such as proteins, nucleic acids, antigens and haptens.

Given that Nu is -NH₂, examples of suitable compounds for forming the present conjugates include those compounds which contain functional groups capable of binding with -NH₂, such as: (1) carboxylate groups, as in, e.g., folic acid, carboxylated vitamin B₁₂, vitamin B₁₂-hemisuccinate at the ribose moiety, N-hemisuccinates of T₄-methyl ester and T₃ methyl ester, thromboxane B₂, carboxypropyl-theophilline, penicillins, cortisol-3-carboxylmethyloxime, estradiol-6-carboxymethyloxime and morphine-6-hemisuccinate; (2) ketone groups, as in, e.g., 3-ketodigoxigenine; (3) aldehyde groups, as in, e.g., digoxin-dialdehyde and bromouridine dialdehyde; (4) halides, as in, e.g., dinitrofluorobenzene and chlorotriazine derivatives of haptens and proteins; (5) active esters, as in, e.g., N-hydroxysuccinimide and imidate derivatives of haptens and proteins; (6) isocyanate and thioisocyanate, as in, e.g., hapten and protein derivatives.

### Brief Description of Drawings

Figures 1-6 are profiles of the separation of mixtures containing carboxylated Vitamin B₁₂, using HPLC and are discussed in Example 5. Figures 7-13 represent the assay performance in competitive immunoassays of different conjugates (tracers) formed between analytes and nucleophilic polysubstituted aryl acridinium esters. These Figures are discussed in Example 6 and 12-16.
Fig. 1 is a diagram showing the separation profile of a mixture of carboxylated vitamin B₁₂ (according to Example 5).
Fig. 2 is a diagram showing the separation profile of another mixture of carboxylated vitamin B₁₂ (run 2 of Example 5).
Fig. 3 is a diagram showing the separation profile of still another mixture of carboxylated vitamin B₁₂ (run 3 of Example 5).
Fig. 4 is yet another diagram of a separation mixture of carboxylated vitamin B₁₂ (run 4 of Example 5).
Fig. 5a is a peak diagram showing peaks in various separation stages of the dicarboxylated vitamin B₁₂ from Figure 2.
Fig. 5b to 5f are peak diagrams showing the analytical HPLC profile of the separated peaks 1 to 5 of Figure 2.
Fig. 6 is a peak diagram showing the analytical HPLC profile of a mixture of monocarboxylated vitamin B₁₂ (from Example 5).
Fig. 7 is a diagram showing the tracer activity of several conjugates of B₁₂-ED-DMAE (according to Example 6).
Fig. 8 is a diagram showing a plot of RLU values against the vitamin B₁₂ concentration (in an assay according to Example 12).
Fig. 9 is a diagram of the RLU values plotted against vitamin B₁₂ concentration (in an assay according to Example 12).
Fig. 10 is a diagram showing the RLU values plotted against the folate concentration in a folate assay (according to Example 13).
Fig.11 is a functional diagram of a standard curve illustrating the displacement of tracer bound to PMP (in cortisol assay according to Example 14).
Fig.12 is a functional diagram showing a standard curve illustrating the displacement of tracer bound to PMP in estradiol assay (according to Example 15).
Fig.13 is a diagram showing a standard curve illustrating the displacement of tracer bound to PMP (in an thromboxane B₂ assay according to Example 16).

The term "activation" (or activate) for the purposes of the specification and the claims means a modification of an existing functional group of a particular compound, which modification generates (or introduces) a new reactive functional group from the prior existing functional group, which new reactive functional group is capable of binding to a target functional group of a second compound. For example, the carboxylic group (-COOH) in thromboxane B₂ (see structure below) can be "activated" to produce a mixed anhydride group 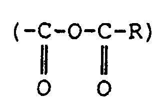 using known procedures in the art. The mixed anhydride can then react with the amino group (-NH₂), for example, of 2',6'-dimethyl-4'-[N-(2 aminoethyl)carbomoyl]phenyl 10-methylacridinium-9-carboxylate bromide (DMAE-ED), to form an amide linkage 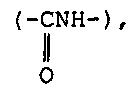 resulting in the formation of a thromboxane B₂-DMAE-ED conjugate (see Example 11 below). As an additional example, the free amino group (-NH₂) group on the surface of alkyl siloxane-coated paramagnetic particles (PMP) (Advanced Magnetics Inc., Cambridge, MA) can be "activated" by derivatization with homobifunctional glutaraldehyde 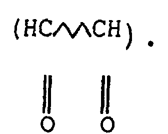 One reactive aldehyde group of the glutaraldehyde covalently binds to the PMP by formation of a Schiff base with the free amino group. The second reactive aldehyde group can then bind with a protein.

The preparation of the conjugates of this invention will vary depending on the acridinium ester and conjugating compound chosen. For example, the following discussion will highlight certain exemplary approaches to forming conjugates from certain compounds and the amino Nu group on the acridinium esters of this invention: (1) given that Nu is -NH₂ and the conjugating compound contains a carboxylic group, the carboxylic acid group is first activated to form an active ester, such as N-hydroxysuccinimide ester, mixed anhydride, or acyl halide. The activated compound is then reacted with the acridinium ester to form the conjugate; (2) given that Nu is -NH₂ and the conjugating compound contains a ketone or aldehyde group, the acridinium ester can be directly reacted with the compound to form a Schiff base. The conjugate can then be reacted with a hydride reducing agent, such as sodium cyanoborohydride, to stabilize the linkage; and (3) given that Nu is -NH₂, the acridinium ester can be reacted directly with a conjugating compound containing a reactive group like halide, isocyanate, or thioisocyanate.

It will be appreciated that the discussion above is not exhaustive and that numerous other conjugates can be formed from the novel acridinium esters of this invention using known procedures in the art.

The conjugates of this invention are useful as luminescent tracers. The conjugates are particularly useful in luminescent assays using specific binding phenomena such as antibody/antigen immunological reactions, nucleic acid hybridization reactions, or ligand/binding protein interactions.

In one embodiment of the present invention, conjugates are prepared using the acridinium esters of this invention and folate or folate derivatives. Preferably, the acridinium ester used contains both nucleophilic and hydrophilic groups. Preparation of this folate-acridinium ester conjugate involves incubating the acridinium ester with a protected folate intermediate of the following formula (which has been activated at one or both of its carboxylic groups): 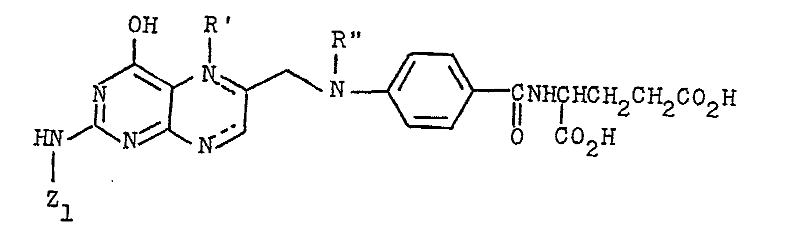 wherein R' is an optional branched or straight-chain, saturated or unsaturated, alkyl of from 1 to 24 carbon atoms, containing 0-20 heteroatoms, preferably nitrogen, oxygen, phosphorous, or sulfur. R" is Z₂, hydrogen, or a branched or straight-chain, saturated or unsaturated, alkyl of from 1 to 24 carbon atoms, containing 0-20 heteroatoms, preferably nitrogen, oxygen, phosphorous, or sulfur. The dotted lines are optional double bonds.

Z₁ and Z₂ are protecting groups. Z₂ is optional. The protecting groups can be any group which can protect the primary and secondary amines from reacting with the activated carboxylic group of the folate either intra- or inter-molecularly. The protecting groups must be removable under conditions which do not deleteriously affect the acridinium ester, preferably in an acidic environment. Useful protecting groups include trifluoroacetyl or t-butyloxycarbonyl groups.

After conjugation of the protected folate intermediate with the acridinium ester, the folate moiety is deblocked by removal of the protecting groups. This deblocking is preferably conducted in an acidic environment using an acidic media such as HBr/acetic acid, which is capable of removing the protecting groups without destroying the integrity of the conjugate. The conjugate so formed can then be used as a tracer in an assay for measuring folates.

In another embodiment of this invention, conjugates are formed using the acridinium esters of this invention and Vitamin B₁₂ (cyanocobalamin). Vitamin B₁₂ has the following structure:

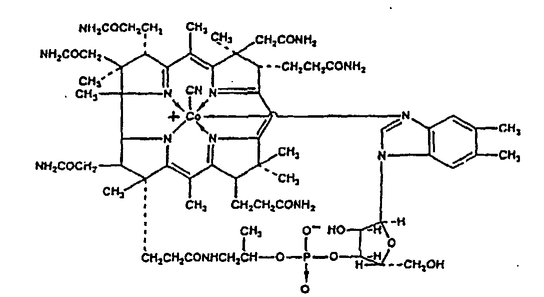

Treating Vitamin B₁₂ with dilute acid will deaminate 1, 2 and/or 3 of the primary propanamide side chains of the Corrin ring to generate a carboxylic function. This carboxylate function is then used to conjugate the Vitamin B₁₂ to the nucleophilic acridinium esters of this invention.

The ratio of monocarboxylic Vitamin B₁₂ (one carboxylate function), dicarboxylic Vitamin B₁₂ (two carboxylate functions), and tricarboxylic Vitamin B₁₂ (three carboxylate functions) will depend on the acid concentration and the reaction time. Monocarboxylic Vitamin B₁₂ is the desired product for the purpose of preparing the conjugates of this invention. Typically, by optimizing known procedures, such as the procedure described in Allen et al, J. Biol. Chem. 247, 7695 (1972), mixtures of mono-, di-, and tricarboxylic Vitamin B₁₂ can be generated which contain up to 40% monocarboxylic Vitamin B₁₂ (see Figures 3 and 4). Prior art procedures utilize a strong anion exchanger to separate the monocarboxylic Vitamin B₁₂ from the di- and tricarboxylic Vitamin B₁₂. [Allen et al, J. Biol. Chem. 247, 7695, 1972.)

There are potentially three forms of monocarboxylic Vitamin B₁₂, depending on which of the 3 primary propanamide side chains have been deaminated. It is desirable to separate these three monocarboxylic Vitamin B₁₂ forms.

It has been unexpectedly discovered that by separating the mixture of carboxylated Vitamin B₁₂ on Reverse Phase High Perfomance Liquid Chromatography (HPLC), the monocarboxylic Vitamin B₁₂ forms can be separated and isolated from the di- and tri-carboxylic forms and from each other. Accordingly, this allows the artisan to obtain individual monocarboxylic Vitamin B₁₂ forms with high purity without the preliminary ion-exchange fractionation step of the prior art.

It has also been unexpectedly discovered that one monocarboxylic Vitamin B₁₂ form is more effective than the other two forms in the conjugates of this invention for use in Vitamin B₁₂ assays.

In a further embodiment of this invention, conjugates are formed using the acridinium esters of this invention and estradiol. These conjugates can be used, for example, as tracers in assays for 17-beta-estradiol.

17-beta-estradiol has the following structure: 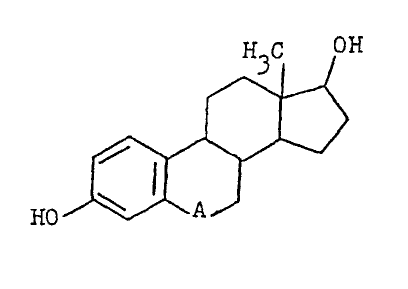 wherein A is CH₂. Useful derivatives of 17-beta-estradiol include 6-keto-17-beta-estradiol 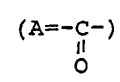 and, preferably, 6-carboxylmethyloxime-17-beta-estradiol

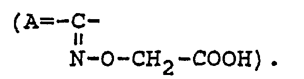

Conjugation with an appropriate acridinium ester of this invention can occur at any of the available functional groups on the 17-beta-estradiol or derivative therefore, i.e., the phenolic 3-OH group, the secondary 17-OH group, or the keto or the carboxymethyloxime group created at the C-6 position. The choice of functional group for conjugation will depend generally on such factors as compatibility with the desired immunoassay system, the stability of conjugate prepared, and the ease of preparation. Preferably the conjugate is prepared by activating the carboxylic group of 6-carboxymethyloxime-17-beta-estradiol and then reacting the activated estradiol derivative with an appropriate acridinium ester of the invention. The resulting conjugate can then be used as a tracer in an assay for determining 17-beta-estradiol.

The acridinium esters of this invention can also be used to form useful conjugates with cortisol. Cortisol has the following structure: 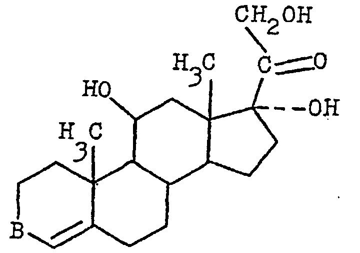 wherein B is 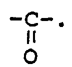 A derivative of cortisol is 3-carboxylmethyloxime cortisol

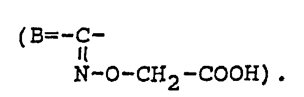

Conjugation with an appropriate acridinium ester of this invention can occur at any of the available functional groups on the cortisol or derivative thereof, i.e., the 21-OH group, the 17-OH group, the 11-OH group, the 20-keto group, the 3-keto group, and the 3-carboxymethyloxime group. Preferably, the conjugate is prepared by activating the carboxylic group of the 3-carboxymethyloxime cortisol and then reacting the activated cortisol derivative with an appropriate acridinium ester of this invention. The resulting tracer can then be used as a tracer in an assay for cortisol.

Useful conjugates can be formed between the acridinium esters of this invention and thromboxane B₂ and other prostaglandin analogs. Thromboxane B₂ has the following structure:

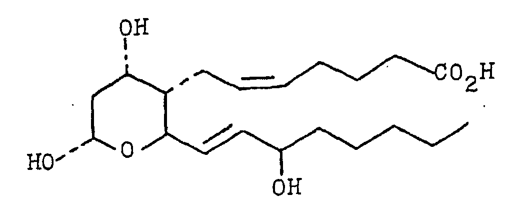

Conjugation with an appropriate acridinium ester of this invention can occur at any of the three hydroxyl groups or the olefin functional groups. The choice of functional group will depend generally on such factors as compatibility with a specific binding protein, and low cross reactivity with non-target prostaglandin analogs. Preferably, the conjugate is prepared by activating the terminal carboxylic group of the thromboxane B₂ and then reacting the resulting thromboxane B₂ derivative with an appropriate acridinium ester of this invention. The resulting conjugate can then be used as a tracer in assays for thromboxane B₂.

This invention also relates to assays utilizing the conjugates of this invention as chemiluminescent tracer compounds. The assays can be homogeneous or heterogeneous. The assays can be competitive inhibition assays where, for example, the analyte to be determined is a univalent hapten or molecule. The assays can also be non-competititve, such as sandwich assays where, for example, the acridinium esters of this invention are conjugated to an antibody or a receptor molecule. The components or reagents of the assays utilizing the conjugates of this invention can be mixed together in any desired manner or sequence provided that the resultant acridinium ester label can be measured in a subsequent detection system. Accordingly, the assays utilizing the conjugates of this invention can be conducted in a forward mode, reverse mode, or a simultaneous mode (see, e.g., U.S. Patents Nos. 4,098,876 and 4,244,940).

Assays for the detection and measurement of Vitamin B₁₂ and folate are illustrative of the assays which can be conducted using the conjugates of this invention. Such assays can use the Vitamin B₁₂-acridinium ester or the folate-acridinium ester conjugates of this invention. A general discussion of radioisotope dilution assays for Vitamin B₁₂ and for folate is found in U.S. Patent No. 4,451,571, herein incorporated by reference.

Assays for the detection or measurement of Vitamin B₁₂ or folate in a sample generally require a sample preparation step wherein the Vitamin B₁₂ or folate in the sample is released (liberated) from endogenous binding proteins. Methods to release the Vitamin B₁₂ or folate from the respective binding proteins include heating or boiling the sample, or using a chemical releasing agent. Typical releasing agents comprise a strong base, such as NaOH. A sulfhydryl compound, such as dithiothreitol (DTT) or beta-mercaptoethanol, is also typically added during the sample preparation step. The sulfhydryl compound can be added before, after, or along with, the addition of the releasing agent.

In one assay for Vitamin B₁₂, following the sample preparation step, the Vitamin B₁₂ tracer compound is combined with the sample and purified hog intrinsic factor (HIF) immobilized on a solid phase. The sample and tracer compound compete for binding sites on the HIF. The amount of tracer compound bound to HIF is inversely proportional to the amount of Vitamin B₁₂ in the sample.

In one assay for folate, following the sample preparation step, the folate tracer compound is combined with the sample and bovine lactoglobulin or folate binding protein (FBP), immobilized on a solid phase. The sample and tracer compound compete for binding sites on the FBP. The amount of tracer compound bound to FBP is inversely proportional to the amount of folate in the sample.

It has been discovered that the sulfhydryl compounds used in the sample preparation step remain in the solid phase at the time of counting (i.e., measuring the amount of label associated with the solid phase). The presence of varying concentrations of the sulfhydryl compound (particularly DTT) in the solid phase can quench the photon output of the chemiluminescent reaction of the acridinium ester label and result in poor assay precision and reduced signal. It was unexpectedly discovered that by incubating the solid phase in a solution comprising a thiol-reactive compound, such as ethyl maleimide, prior to counting, the quenching effect of the sulfhydryl compound is reduced or eliminated. Preferably, the concentration of the thiol-reactive compound in the solution is about 0.01mM to about 50mM, more preferably about 0.5mM to about 10mM, and most preferably, about 1mM.

The following Examples illustrate the present invention.

### Example 1

### Preparation of 2', 6'-Dimethyl-4'-[N-(2-aminoethyl)carbomoyl]phenyl 10-methylacridinium-9-carboxylate bromide (DMAE-ED)

A solution of 2', 6'-dimethyl-4'-carboxylphenyl 10-methylacridinium-9-carboxylate bromide (DMAE, 200mg, 0.43mmole) (see copending U.S. Application Serial No. 226,639, filed on August 1, 1988) in 30ml of dimethylformamide (DMF) was cooled in ice bath, treated with triethylamine (0.25ml, 1.72mmole), ethylchloroformate (0.08ml, 0.85mmole) and 30ml of chloroform to form a reaction mixture. After 20 min. of stirring, the reaction mixture was transferred to a dried dropping funnel and added dropwise over a 15 minute period to a solution of ethylenediamine in 10ml of DMF/CHCl₃ (1:1) to form a second reaction mixture.

The second reaction mixture was then stirred at room temperature overnight and then evaporated to dryness under vacuo. The residue produced from the evaporation was taken up in 3-4ml of chloroform/methanol/water (65:25:4), purified on two 20x20cm preparative thin layer chromatography TLC plates (Silica gel 60, F254, Merck & Co., Inc., Rahway, NJ) and developed with the same solvent system. The yellow major band which developed (Rf = 0.47) (which could also be detected under long and short UV light) was stripped and eluted with the same solvent system. The eluent was then evaporated. The residue from this evaporation was triturated with 30ml of 10% methanol/chloroform and filtered through Whatman #1 filter paper under gravity. The filtrate so produced was evaporated to produce DMAE-ED (110mg, 50%). Fast Atom Bombardment (FAB) Mass Spectral Analysis (performed by Oneida Research Services, Whitesboro, N.Y.) in the positive ion mode gave a M+ peak of 428. Isotopic bromide peaks 79 and 81 of about equal intensity were detected in the negative ion mode.

### Example 2

### Preparation of N-tert-Butyloxycarbonyl-S-(3-sulfopropyl)cysteine (BOC-SulfoCys)

2-(tert-butoxycarbonyloxyimino)-2-phenylacetonitrile (BOC-ON) (121mg, 0.49mmole) (Aldrich Chem. Co., Milwaukee, Wisconsin) was added to a solution of S-(3-sulfopropyl)cysteine (SulfoCys, 100 mg, 0.41 mmole), (prepared by the method of U.T. Ruegg and J. Rudinger, J. Peptide Protein Res. 6, 447, 1974), and triethylamine (0.18ml, 1.23mmole) in 3ml of 50% aqueous dioxane to form a reaction mixture. The reaction mixture was heated at 50°-55°C for 1 hour to obtain a yellow solution. The reaction mixture was then cooled, diluted with 5ml of water, and washed with ethylacetate (3x10ml). The resultant aqueous layer was evaporated to dryness under vacuo by coevaporating with methanol twice. TLC (Silica gel 60, Merck & Co., Inc.) analysis of the residue produced by the evaporation, using a solvent system of chloroform/methanol/water (55:40:5), showed complete conversion of SulfoCys to BOC-SulfoCys.

### Example 3

### Preparation of 2', 6'-Dimethyl-4'-{N-[N-(2-tert-butyloxycarbonylamino-3-S-(3'-sulfopropyl)-thiopropionyl)-2-amino ethyl]carbamoyl}phenyl 10-methylacridinium-9-carboxylate bromide (BOC-SulfoCys-ED-DMAE)

A solution of BOC-SulfoCys (3.42mmole) (Example 2), and DMAE-ED (400mg, 0.79mmole) (Example 1) in 110ml of DMF/CHCl₃ (1:1) was treated with dicyclohexylcarbodiimide (325mg, 1.57mmole), stirred at room temperature for 3 hours and evaporated to dryness. The residue from the evaporation was taken up in about 15ml of chloroform/methanol/water (65:25:4) and purified on 8 preparative TLC plates (Silica gel 60, Merck & Co., Inc.) developed with the same solvent system.

The major yellow band which developed at about Rf of 0.55 was stripped and eluted with the same solvent system. The eluent was then evaporated to dryness under vacuo to produce BOC-SulfoCys-ED-DMAE (630mg, 90%)

### Example 4

### Preparation of 2',6'-Dimethyl-4'-{N-[N-(2-amino-3-S-(3'-sulfopropyl)-thiopropionyl)-2-aminoethyl]carbamoyl}phenyl 10-methylacridinium-9-carboxylate bromide (SulfoCys-ED-DMAE)

A solution of BOC-SulfoCys-ED-DMAE (630mg, 0.715mmole) (Example 3) in 4ml of 36% HBr/Acetic acid was kept at room temperature for 5 hours to form a reaction mixture. The reaction mixture was added dropwise to about 30ml of anhydrous ethylether, forming a gummy precipitate and supernatant. The supernatant was removed from the precipitate. The precipitate was then dissolved in about 10ml of methanol and the resultant solution was then added dropwise to about 30ml of fresh anhydrous ethylether, forming a yellow precipitate and supernatant. The yellow precipitate and supernatant were then filtered through a medium porosity frit and the resultant yellow solid residue was then washed with anhydrous ethylether, and then air dried to produce SulfoCys-ED-DMAE (438mg, 93.7%).

FAB Mass Spectral analysis (performed by both Oneida Research Services, Whitesboro, N.Y. and Institute of Chemical Analysis, Northeastern University, Boston, MA) in the positive ion mode gave a M+ peak of 653.

### Example 5

### Preparation of Monocarboxylic Vitamin B₁₂

### A. Preparation of Deaminated Vitamin B₁₂

Vitamin B₁₂ (1.0g, 0.738mmole) (Sigma Chemicals, St. Louis, MO) was added to 80ml of 0.5N HCl to form a reaction mixture and stirred at room temperature for 65 hours. The reaction mixture was then loaded onto a 4x15cm column of Bio-Rad AG1-X8 (acetate form) (Bio-Rad Laboratories, Richmond, CA), 100-200 mesh, packed and eluted as described in Allen, R.H. and Majerus, P.W., J. Biological Chem., 247, 7695-7701 (1972). The initial 300ml of red eluent was collected and evaporated to dryness under vacuum to produce about 1 gram of a mixture of mono-, di-, and tricarboxylated Vitamin B₁₂.

### B. Preparation of Mixture of Mono-carboxylated Vitamin B₁₂

The mixture of carboxylated Vitamin B₁₂ prepared in A was fractionated by QAE-Sephadex A-25 chromotography to obtain a mixture of monocarboxylated Vitamin B₁₂ as described by Allen et al. (J. Biol. Chem. 247, 7695, 1972).

### C. Preparative HPLC for the Separation of the Carboxylated Vitamin B₁₂

50mg of the mixture of the mono-, di-, and tri-carboxylated Vitamin B₁₂ from A in 2ml of water was injected into a Waters Delta-Prep 3000 HPLC system (Waters Associates, Milford, MA) with an ISCO-Foxy fraction collector, an ISCO-2150 peak separator (ISCO, Lincoln, Nebraska) and a YMC AP 363-15, 30mm x 25cm stainless steel column packed with C18-bonded silica of 15um particles, spherical shape, and 300A pore size (YMC, Inc., Morris Plains, N.J.).

The carboxylated Vitamin B₁₂ was eluted from the column for each run using acetonitrile as Solvent B and 0.05M triethylammonium acetate, pH 4.5, as Solvent A, in the following manner:
1. Run 1 - step-gradient elution:
   20 min. on 8% Solvent B/92% Solvent A then
   10 min. on 10% Solvent B/90% Solvent A then
   50 min. on 15% Solvent B/85% Solvent A
2. Run 2 - isocratic elation:
   Using 15% Solvent B/85% Solvent A
3. Run 3 - step-gradient elution:
   10 min. on 10% Solvent B/90% Solvent A
   10 min. on 15% Solvent B/85% Solvent A
4. Run 4 - isociatic elution:
   10% Solvent B/90% Solvent A
The flow rate of the column was 20ml/min. for each run and the eluted materials were detected at a wavelength of 280nm.

Figure 1 is a profile of the separation of the mixture of carboxylated Vitamin B₁₂ obtained in Run 1. Five peaks were collected although Peak 5 was not recorded because the preprogrammed recording time ended prior to elution of Peak 5. Peak 5 was determined based on the characteristic red color of the fraction collected. Peak 5 was then added to the graph as a dotted line peak indicating its location had it been recorded.

Figures 2-4 are profiles of the separation of the mixture of carboxylated Vitamin B₁₂ obtained in Runs 2, 3, and 4, respectively.

These results demonstrate that with a properly chosen solvent system and preparative HPLC column, the separation profile of the mixture of the carboxylated Vitamin B₁₂ can be adjusted. For example, as seen in Figure 2 and Figure 5A which show the profile of the analytical HPLC of the same mixture of carboxylated Vitamin B₁₂ (See D below), Peak 3 of fig. 5A (retention time of 13.84 min.) was split into peaks 3 and 4 (Fig. 2) using the preparative column.

### D. Analytical HPLC Profile of the Carboxylated Vitamin B₁₂ Derivatives

5 to 20ug of the mixtures of carboxylated Vitamin B₁₂ prepared in A, B, and Run 2 of C, in 20ul of water were each injected into a Beckman 344 HPLC system (Beckman Instruments, San Ramon, CA). The HPLC system contained a 3.9mm x 30cm stainless steel column packed with uBondapak C18 10um particles with irregular shape and 120A pore size (Waters Associates, Milford, MA). The mixtures were each eluted isocraticly from the column using 10% acetonitrile and 90% 0.05M triethylammonium acetate, pH4.5. The flow rate of the elution was 1.5ml/min. and the eluted materials were detected at a wavelength of 280nm.

Figure 5A is a profile of the separation of the carboxylated Vitamin B₁₂ from A. Peak 1 represents the unreacted Vitamin B₁₂, Peak 2 (retention time of 11.97 min.) and peak 3 (retention time of 13.84 min.) represent the 3 incompletely separated monocarboxylated Vitamin B₁₂ forms present in the mixture. Peak 5 in Figure 5A probably represents the dicarboxylated Vitamin B₁₂ form.

Figures 5B-5F are the analytical HPLC profiles of the separated peaks 1-5 (Figure 2) obtained in Run 4 of C. Figure 5D and Figure 5E show the close retention times (13.54 min. and 14.33 min., respectively) of the two different monocarboxylated Vitamin B₁₂ forms which comprised peak 3 of Figure 5A and peaks 3 and 4 of Figure 2.

Figure 6 is the analytical HPLC profile of the mixture of the 3 monocarboxylated Vitamin B₁₂ forms from B. Peaks 1 and 2 of Figure 6 have nearly the same retention times as Peaks 2 and 3 of Figure 5A.

### Example 6

### Preparation of Conjugates from Monocarboxylic Vitamin B₁₂ and DMAE-ED (B₁₂-ED-DMAE)

Using the 3 monocarboxylic Vitamin B₁₂ forms prepared and isolated in Example 5 (designated hereinafter as monocarboxylic Vitamin B₁₂ forms I, II, and III, which have retention times of 11.58, 13.54, and 14.33 min., respectively) and DMAE-ED prepared in Example 1, three separate but similar conjugating reactions were carried out to prepare the tracers as following:

A solution of monocarboxylic Vitamin B₁₂ form I (10mg., 7.4umole) in 1.8ml of DMF was cooled in ice bath, treated with triethylamine (10.5ul, 74umole, in 100ul DMF) and ethyl chloroformate (2.8ul, 30umole, in 100ul DMF) to form a reaction mixture. After stirring for 30 minutes, the reaction mixture was evaporated to dryness to remove the excess ethyl chloroformate to produce a residue. DMAE-ED (3.4mg, 6.7umole) and triethylamine (5.2ul, 37umole) in 2ml of DMF, were added to the residue to form a second reaction mixture. The second reaction mixture was stirred at room temperature overnight and then evaporated to dryness under vacuo. The crude products so obtained were purified on one analytical silica gel 20x20cm TLC plate (Silica gel 60, Merck & Co, Inc.), developed with chloroform/methanol/water (55:40:5).

Two red bands (hereinafter referred to as the upper and the lower bands) which developed between Rf of 0.47-0.57 were each separately stripped and eluted with the same solvent system. Each eluent was evaporated to dryness to produce a B₁₂-ED-DMAE tracer.

The same procedure described above was repeated for monocarboxylic Vitamin B₁₂ forms II and III. As a result, from the 3 monocarboxylic Vitamin B₁₂ forms, a total of six B₁₂-ED-DMAE conjugates (designated 1 through 6) were isolated. Conjugates 1 and 2 were prepared from form I, 3 and 4 were prepared from form II, and 5 and 6 were prepared from form III. The conjugates were each diluted in phosphate buffered saline (PBS) with 0.1% bovine serum albumin (BSA) and were simultaneously screened for tracer activity (Figure 7) using the following procedure:

A series of Vitamin B₁₂ standards in 1% human serum albumin (HSA) (in 120mM PBS containing 0.2% sodium azide and 0.4g/l merthiolate) were treated by adding 1/20 volume of 1.35M DTT, to produce treated standards. 100ul of each treated standard was added to a 12x75mm plastic tube. To each tube was then added 100ul of 0.5N NaOH and 0.5ml of an IF-PMP (100ug) (prepared as described in Example 12A below except that the suspension contained 3ug IF/g PMP, the heat stress step was omitted, and the IF-PMP was resuspended in 0.16M boric acid, 10mM phosphate, 0.127M NaCl, and 0.1% sodium azide, pH 7.0). 100ul of PBS/BSA containing 12-29x10⁶ relative light units (RLU) (1 RLU = 1 photon count) of the B₁₂-ED-DMAE conjugate to be tested, was added to each tube and the tubes were then incubated at room temperature for one hour. The solid phase in each tube was magnetically separated from the supernatant and the supernatant was then decanted. The solid phase in each tube was washed once with 1ml of water. The solid phase was then resuspended in 100ul water and counted as described in Example 12B below. Table 1 shows the results obtained. In Table 1 T represents the total RLU of each conjugate added, Bₒ represents the total RLU associated with the solid phase in the final resuspension, for each conjugate, in the absence of any Vitamin B₁₂, end Bₒ/T is the percentage of the total RLU added which were associated with the solid phase, for each conjugate.

**Table 1**

| Conjugate | T | Bₒ | Bₒ/T % |
|---|---|---|---|
| 1 | 18x10⁶ | 3.8x10⁴ | 0.21 |
| 2 | 29x10⁶ | 5x10⁴ | 0.17 |
| 3 | 17.5x10⁶ | 7.7x10⁴ | 0.44 |
| 4 | 15.2x10⁶ | 6.6x10⁴ | 0.44 |
| 5 | 20.6x10⁶ | 26.1x10⁴ | 1.27 |
| 6 | 12x10⁶ | 20x10⁴ | 1.67 |

Figure 7 is a plot of B/Bₒ against Vitamin B₁₂ concentration for each of the conjugates. B represents the total RLU associated with the solid phase in the final resuspension for a particular concentration of Vitamin B₁₂ in the sample and Bₒ is as described above. The conjugate that performed the best (highest Bo/T value) came from the lower band (conjugate #6) which originated from monocarboxylic Vitamin B₁₂ III.

100ug of the B₁₂-ED-DMAE conjugate #6 was separated on an analytical HPLC system as described in Example 5D eluted with mixture of 0.05M triethylammonium acetate, pH 4.5 (Solvent A) and acetonitrile (Solvent B) in linear gradient from 40% B/60% A to 50% B/50% A over a 10 min. period. The flow rate of the eluent was 1 ml/min. and the eluted materials were detected at 260nm. The chromatogram revealed the presence of two peaks (retention times of 5.66 min. and 7.86 min.). These two peaks when isolated and evaluated separately gave identical assay performance whether combined or alone.

### Example 7

### Preparation of N -Trifluoroacetyl -Folic Acid (TFA-Fol)

A mixture of folic acid (1.0g, 2.27mmole) and trifluoroacetic anhydride (2ml, 6.4mmole) was stirred at room temperature for 1 hour and then evaporated under vacuo. The residue from the evaporation was triturated with a minimal amount of methanol and the supernatant removed by filtration. The resultant wet cake was evaporated to dryness to produce TFA-Fol with an Rf of 0.35 when chromotographed on a TLC plate (Silica gel 60, Merck & Co.) using chloroform/methanol/water (55:40:5).

### Example 8

### Preparation of Folate-SulfoCys-ED-DMAE conjugate

A solution of TFA-Fol (27mg, 0.05mmole) (Example 7) in 3.75ml of DMF was diluted with 1.8ml of chloroform, cooled in an ice bath and treated with triethylamine (0.065ml, 0.45mmole) and ethyl chloroformate (0.03ml, 0.3mmole) to form a reaction mixture. After 30 min. of stirring, the reaction mixture was evaporated to dryness under vacuo. To the residue which resulted from the evaporation were added 4.5ml of DMF/chloroform (2:1), SulfoCys-ED-DMAE (31mg, 0.04mmole) and triethylamine (0.035ml, 0.24mmole) to form a second reaction mixture. The second reaction mixture was stirred at room temperature overnight and evaporated to form a second residue.

The second residue was purified on a 10x20cm silica gel preparative TLC plate (Silica gel 60, Merck & Co.) developed with chloroform/methanol/water (55:40:5).

The yellow band which developed at about an Rf of 0.64 was stripped and eluted with the same solvent system. The eluent was evaporated to produce crude TFA-Fol-SulfoCys-ED-DMAE (11 mg). TLC analysis of the product showed two major UV positive spots (Rf of 0.6 and 0.5). The spot having Rf of 0.5 was not affected by the subsequent deblocking conditions and was, therefore, considered as an undesirable contaminant.

The crude TFA-Fol-SulfoCys-ED-DMAE obtained above was treated with 200ul of 36% HBr/Acetic acid at room temperature overnight to form a third reaction mixture. This third reaction mixture was treated with about 10ml of anhydrous ethylether to form a precipitate. After 1 hour of standing, the supernatant was removed from the precipitate by careful pipeting. The precipitate was then dissolved in about 0.5ml of chloroform/methanol/water (65:25:4) and purified on one 20x20cm silica gel analytical TLC plate (Silica gel 60, Merck & Co., Inc.). The TLC plate was developed with chloroform/methanol/water (55:40:5). The yellow band which developed at Rf of 0.38 was stripped and eluted with the same developing solvent system. The eluent was evaporated to produce the Folate-SulfoCys-ED-DMAE conjugate.

### Example 9

### Preparation of Cortisol -3 -CMO -ED -DMAE Conjugate

A solution of 3-carboxylmethyloxime-cortisol (Cortisol-3-CMO, 10mg, 0.022mmole) (Steraloids, Wilton, N.H.) in 0.2ml of DMF was diluted with 0.8ml of chloroform, cooled in an ice bath, and treated with dicyclohexylcarbodiimide (DCC, 5.5mg, 0.0266mmole) in 0.2ml of chloroform to produce a reaction mixture. After 10 min. of stirring, the reaction mixture was treated with a solution of DMAE-ED (5.5mg, 0.01mmole) in 0.4ml of DMF to produce a second reaction mixture. The second reaction mixture was stirred at room temperature overnight and evaporated under vacuo. The residue from the evaporation was purified on a 10x20cm silica gel preparative TLC plate (Merck & Co.) and developed with 10% methanol/chloroform. The yellow band which developed at Rf of 0.28 was stripped and eluted with 20% methanol/chloroform. The eluent was evaporated to give Cortisol-3-CMO-ED-DMAE (3.56mg, 42%). FAB Mass Spectral analysis (performed by Institute of Chemical Analysis, Northeastern Univ., Boston, MA) in the positive ion mode gave a M+ peak of 845.

### Example 10

### Preparation of Estradiol-6-CMO-ED-DMAE Conjugate

A solution of 6-carboxymethyloxime-17-beta-estradiol (Estradiol-6-CMO) (23.1mg, 0.062mmole) (Steraloids, Wilton, N.H.) in 2ml of DMF/CHCl₃ (1:1) was cooled in an ice bath, and treated with DCC (15.4mg, 0.074mmole) to produce a reaction mixture. After 10 min. of stirring, the reaction mixture was treated with DMAE-ED (30.1mg, 0.059mmole) to produce a second reaction mixture. The second reaction mixture was stirred at room temperature overnight and evaporated under vacuo. The residue from the evaporation was purified on one 20x20cm silica gel preparative TLC plate developed with 5% methanol/chloroform. The yellow band which developed at Rf of 0.17 was stripped and eluted with 20% methanol/chloroform. The eluent was evaporated to give Estradiol-6-CMO-ED-DMAE (11.9mg, 26%). FAB Mass Spectral analysis (performed by Institute of Chemical Analysis, Northeastern University, Boston, MA) in the positive ion mode gave a M+ peak of 789.

### Example 11

### Preparation of Thromboxane B₂-ED-DMAE Conjugate (TxB₂-ED-DMAE)

A solution of Thromboxane B₂ (TxB2) (2.5mg, 0.0067mmole) (Biomol, Plymouth Meeting, PA) in 0.4 ml of DMF/CHCl₃ (1:1) was cooled in an ice bath, treated with triethylamine (6ul, 0.04mmole), and ethyl chloroformate (2ul, 0.02mmole) to produce a reaction mixture. After 30 min. of stirring, the reactionmixture was evaporated to dryness. The residue from the evaporation was dissolved in 0.4ml of DMF/CHCl₃ (1:1), treated with triethylamine (6ul, 0.04mmole) and DMAE-ED (4.5mg, 0.009mmole) to produce a second reaction mixture. The second reaction mixture was stirred at room temperature and evaporated under vacuo to form a second residue. The second residue was taken up in about 0.5ml of chloroform/methanol/water (65:25:4) and purified on a 20x20cm silica gel analytical TLC plate (Merck & Co.) developed with 15% methanol/chloroform.

The major yellow band which developed at Rf of 0.49 was stripped and eluted with 15% methanol/chloroform. The eluent was evaporated to produce TxB₂-ED-DMAE.

### Example 12

### Vitamin B₁₂ Assay

### A. Preparation of Intrinsic Factor Paramagnetic Particles (IF-PMP)

PMP (obtained from Advanced Magnetics Inc., Cambridge, MA) were activated with glutaraldehyde as described in U.S. Patent No. 4,454,083.

To a solution of human serum albumin (HSA) (400mg, Immunosearch, Toms River, N.J.) in 25ml of 10mM sodium phosphate, pH 7.4, was added purified hog Intrinsic Factor (purchased from Dr. R.H. Allen, University of Colorado Medical Center, Denver, CO) (75ug) in 5ml of saline to produce a protein mixture.

The protein mixture was added to a suspension of the activated PMP (5g) in 60ml of 10mM sodium phosphate and shaken at room temperature overnight to produce IF-PMP.

The IF-PMP were then washed and the excessive activated groups were quenched with glycine.

The IF-PMP were resuspended in 200ml of 30mM PBS with 0.1% sodium azide, 0.1% BSA and 0.001% BgG, cured at 50°C for 16 hrs, washed three times with 10mM sodium phosphate, washed three times with Glycine Buffer (0.325 glycine, 0.1% sodium azide, and 0.1% BSA, pH 7.8), resuspended in the Glycine Buffer (25mg/ml) and stored at 4°C until needed.

### B. Simultaneous Assay

A series of standards in 6% HSA (in 120mM PBS with 0.2% sodium azide and 0.4g/l merthiolate) with known increasing amounts of Vitamin B₁₂, were added to 12x75mm plastic tubes (100ul/tube). 0.1ml of Releasing Agent (0.5M NaOH, 50ug/ml KCN, 0.3ug/ml cobinamide, 0.064M dithiothreitol) was added to the tubes and the tubes were incubated at room temperature for 15 minutes. The IF-PMP prepared in A was diluted 1:312 in the Glycine Buffer (80ug/ml). 0.5ml of the diluted IF-PMP (40ug/tube) and 0.1ml of the B₁₂-ED-DMAE conjugate #6 prepared in Example 6, diluted in PBS with 0.1% BSA and 0.1% sodium azide (4x10⁶ RLU/tube), were then added to the tubes and the tubes were incubated for 60 min. at room temperature.

The tubes were then placed in a magnetic rack useful for magnetic separation of paramagnetic particles in tubes (available from Ciba Corning Diagnostics Corp., Medfield, MA). The magnetic field separated the particles from the supernatant and the supernatant was then decanted. The particles were washed once in 1ml of water, vortexed, magnetically separated from the wash and decanted. The particles were then resuspended in 0.1ml of a 1mM ethyl maleimide.

The tubes were then placed in a luminometer (MAGIC^{R} LITE Analyzer, Ciba Corning Diagnostics Corp., Medfield, MA). 0.3ml of a solution of 0.5% hydrogen peroxide in 0.1 N HNO₃ was added to each tube by the luminometer and the light emission was triggered by the addition of 0.3ml of 0.25N NaOH containing ARQUAD surfactant (Armack Chemicals, Chicago, Illinois). The measured RLU's of each tube normalized against the RLU's of the zero standard were plotted against their respective Vitamin B₁₂ concentrations as shown in Figure 8.

### C. Split Incubation Assay

A series of Vitamin B₁₂ standards in 5% HSA (in PBS with 0.2% sodium azide, 2mg/l amphotericin and 24mg/l gentamycin) with known increasing amounts of Vitamin B₁₂ were added to tubes (100ul/tube) and incubated with the Releasing Agent of B except that the cobinamide was omitted. 0.5ml of the diluted IF-PMP of B (40ug/tube), but with 0.06ug/ml cobinamide added to the buffer, was added to each tube and the tubes were then incubated for 45 minutes at room temperature. 0.1ml of the B₁₂-ED-DMAE conjugate #6 (8x10⁶ RLU) prepared in Example 6 diluted with 10mM PBS, pH 7.4, containing 0.1% sodium azide and 0.1% BSA, was then added to each tube and the tubes were then incubated for 30 minutes at room temperature. The particles in the tubes were magnetically separated, washed, resuspended, and counted as described in B. The measured RLU's normalized against the RLU's of the zero standard for each tube were plotted against their respective Vitamin B₁₂ concentration as shown in Figure 9.

### Example 13

### Folate Assay

### A. Reagents

The standards used in the Folate assay were PGA (pteroylglutamic acid) (Sigma Chemical Co, St. Louis, MO) dissolved in 120mM PBS, pH 7.4 with 4% HSA, 0.2% sodium azide, 2mg/l amphotericin, and 24mg/l gentamycin added as preservatives. The folate concentrations were zero, 0.25, 0.5, 1.0, 2.5, 5, 10, 15, 20, and 30ng PGA/ml.

The Releasing Agent was 0.5N NaOH containing 64mM dithiothreitol.

Folate-SulfoCys-ED-DMAE conjugate (8.8x10¹¹ RLU) obtained in Example 8 was first dissolved in 22ml of 10% DMF/water. The solution was then further diluted 1:11250 with 325mM glycine containing 0.1% BSA and 0.1% sodium azide to form a second solution. This second solution was filtered through a 0.2um cellulose acetate filter (Schleicher and Schuell, Keene, N.H.) to produce a tracer solution. 500ul of the tracer solution was added per test.

The binder in the assay was Folate Binding Protein (FBP) (a bovine milk lactoglobulin, purchased from Dr. R.H. Allen, University of Colorado Medical Center, Denver, CO). FBP-PMP and Bovine Gamma Globulin (BgG)-PMP were prepared by the method described in U.S. Patent No. 4,454,088. The FBP-PMP (0.96mg/ml) was diluted 1:60 in 10mM PBS with 0.1% BSA and 0.1% sodium azide, pH 7.4. This was bulked with BgG-PMP at 0.4mg/ml to form the solid phase binder. 100ul of this solid phase binder was added per test, resulting in the addition of 1.6ug FBP-PMP and 40ug BgG PMP. In the final assay there was 100ul of sample or standard, 100ul of Releasing Agent, 500ul of tracer solution and 100ul of solid phase binder, for a total assay volume of 800ul.

### B. Assay Procedure

Standards or samples (100ul) were added to 12x75mm polystyrene tubes (Sarstedt, West Germany). To each tube was added 100ul of the Releasing Agent of A. The tubes were vortexed and incubated for 15 min. at room temperature. The tracer solution of A (500ul) was then added to each tube, followed by the addition of the solid phase binder of A (100ul). The tubes were vortexed again and incubated for one hour at room temperature. The tubes were then put on a magnetic separator for 3 minutes, decanted, and blotted. 1ml of deionized water was added to each tube to wash out excess unbound tracer. The solid phase in the tubes was magnetically separated for 3 min., the supernatant decanted, and the tubes drained for 3 minutes. To the resulting pellets in the tubes was added 100ul of 1mM ethyl maleimide. The tubes were then placed in a luminometer and counted as described in Example 12B. The RLU's for each standard were normalized against the RLU's of the zero standard and plotted against the respective folate concentration of the standards to give a displacement curve as shown in Figure 10.

### Example 14

### Cortisol Assay

### A. Reagent Preparation

The Cortisol-ED-DMAE conjugate of Example 9 was dissolved in methanol and kept at -20°C as a stock solution. The final cortisol-ED-DMAE conjugate was diluted in a buffer containing 10mM sodium phosphate, pH 7.4, 0.1% bovine serum albumin, 0.4mg/ml of 8-anilino-1-naphthalenesulphonate, 0.1% Triton X-100, and 0.05% sodium azide, to produce the tracer solution.

Rabbit anti-cortisol antiserum was bought from Bioclinical Group, Cambridge, MA. The antibody was immobilized on PMP (Advanced Magnetics Inc.) as described in U.S. Patent No. 4,554,088 except that 0.01M sodium acetate buffer, pH 5.5, was used instead of 0.1M sodium phosphate buffer, pH 7.4. The final PMP wet cake was diluted with a buffer containing 0.01M sodium phosphate, 0.1% bovine serum albumin, 4ng/ml 11-deoxycortisol, and 0.4mg/ml 8-anilino-1-naphthalene sulfonate, pH 7.4, to form a PMP suspension (10mg/ml).

### B. Assay Procedure

25ml each of cortisol standards with concentrations from 0 to 750ng/ml were added to 12x75mm polystyrene test tubes (Sarstedt, West Germany) in duplicate. 100ul of the tracer solution of A with total activity of 10⁶ RLU were then added to the tubes followed by 500ul of the diluted PMP suspension of A. After vortexing, the tubes were incubated for 1 hour at room temperature. The PMP in the tubes were magnetically separated from the supernatant. The supernatant in each tube was then decanted and the PMP in each tube were washed once with 500ul of 0.87% saline and then resuspended in 100ul of water. The tubes were then placed in a luminometer and counted as described in Example 12B. A standard curve in Figure 11 shows the displacement of tracer bound to PMP by added cortisol in the standard. The displacement is inversely proportional to the concentration of the cortisol in the standard.

### Example 15

### Estradiol Assay

### A. Reagent Preparation

The Estradiol-ED-DMAE conjugate of Example 10 was dissolved in methanol and kept at -20°C as a stock solution. The stock solution was diluted with 0.01M sodium phosphate buffer, pH 7.4, containing 0.1% bovine serum albumin, 0.15M NaCl, and 0.05% sodium azide to form a tracer solution.

Monoclonal anti-estradiol antibody was produced in mice (A/J) by immunization with a BSA-estradiol conjugate and subsequent fusion of the splenocytes with Sp2/0-Ag14 myeloma cells by the procedure described by Kohler and Milstein in Nature (London), vol. 256, pp. 495-497 (1975). Hybridoma cells secreting anti-estradiol antibody were detected by the following procedure: Supernatant from the cells were diluted 1:5 in phosphate buffered saline containing 1mg/ml bovine serum albumin. 100ul of each diluted supernatant and 100ul of acridinium ester-labelled estradiol-fowl gamma globulin as tracer were added to a test tube and incubated for one hour at room temperature. Goat anti-mouse IgG coupled to paramagnetic particles were added to each tube and incubated further for 10 minutes at room temperature. The particles were magnetically separated and read on a luminometer for tracer bound to the particles. The cells that tested positive (i.e., produce photon counts over background) were plated at 0.1 cell/well and retested after growth.

Cells resulting from this regrowth which tested positive were then injected intraperitoneally into pristane-primed mice (CAF₁). Ascitic fluid from these mice was collected after 3-5 weeks. The anti-estradiol antibody was used directly without further purification.

Goat anti-mouse IgG PMP particles were prepared by immobilizing the IgG fraction of goat anti-mouse IgG antiserum (Jackson Laboratory, PA) on paramagnetic particles by the method described in U.S. Patent No. 4,454,088. The final PMP wet cake was diluted with phosphate buffered saline containing 1mg/ml bovine serum albumin (PBS/BSA)to produce a PMP suspension with a final concentration of 10mg/ml.

### B. Assay Procedure

50ul each of a series of estradiol standards with concentration range from 0 to 2000pg/ml were added to 12x75mm polystyrene test tubes (Sarstedt, West Germany) followed by the addition of 100ul of the tracer solution of A with total activity of 385,000 RLU. 100ul of the ascitic fluid from A was diluted 1:20000 in PBS/BSA buffer and then was added to each tube. The tubes were all vortexed and incubated for one hour at room temperature. The PMP suspension of A was diluted in PBS/BSA to a final concentration of 80ug/ml. 500ul of the diluted PMP suspension was then added to each of the test tubes. The tubes were then vortexed and incubated for 30 minutes at room temperature. The PMP in the tubes were then magnetically separated. The PMP in each tube was then washed once with 500ul of saline containing 0.05% Triton X-100, magnetically separated, and the supernatant decanted. The PMP was then resuspended in 100ul of water. The tubes were then placed in a luminometer and counted as described in Example 12B. The displacement curve in Figure 12 shows that the photon counts are inversely proportional to the concentration of estradiol in the standards.

### Example 16

### Thromboxane B₂ (TxB₂) assay

### A. Reagent preparation

The TxB₂-ED-DMAE conjugate of Example 11 was dissolved in methanol and kept at -80°C as a stock solution. The stock solution was diluted with 0.01M sodium phosphate buffer, pH 7.4, containing 0.1% BSA, 0.15M NaCL and 0.05% sodium azide to produce the tracer solution.

Rabbit anti-TxB₂ antiserum was bought from Cayman Chemicals Co., Ann Arbor, MI. The antiserum was diluted with 0.01M sodium phosphate buffer, pH 7.4, containing 0.15M sodium chloride, 1mg/ml bovine serum albumin, 0.05% sodium azide.

Goat anti-rabbit IgG PMP was prepared by immobilizing the IgG fraction of goat anti-rabbit IgG antiserum (Jackson Laboratory, PA) on paramagnetic particles (PMP) by the method described in U.S. Patent No. 4,454,088. The final PMP wet cake was diluted with PBS/BSA buffer (10mg/ml) to produce a PMP suspension.

### B. Assay procedure

100ul of each of a series of TxB₂ standards in PBS/BSA (0-30ng/ml) were added to polystyrene test tubes (12x75mm, Sarstedt, West Germany). 100ul of the tracer solution of A with a total activity of 350,000 RLU, was then added to each tube. 100ul of rabbit anti-TxB₂ antiserum (prepared in A diluted 1/40000 in PBS/BSA) was pipeted into all tubes. All tubes were vortexed and incubated at room temperature for 1 hour. 500ul of the diluted PMP suspension of A was then added to all the tubes and incubated for 45 minutes at room temperature. The PMP in the tubes were then magnetically separated from the supernatant. The supernatant was decanted and the PMP were then washed once with 500ul of water then resuspended in 100ul of water. The tubes were then placed in a luminometer and counted as described in Example 12B. The displacement curve in Figure 13 shows that the photon counts were inversely proportional to the concentration of TxB₂ in the standards.

## Claims

1. An acridinium ester **characterised in that** it corresponds to the following general formula: 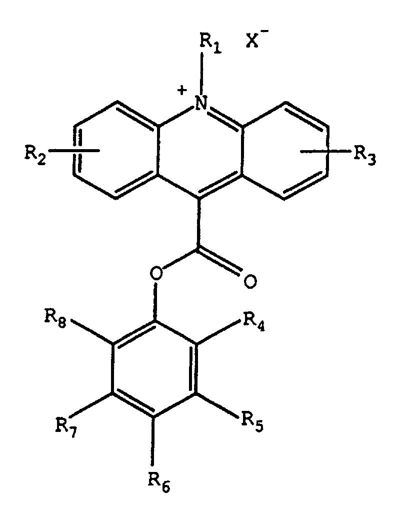 wherein
R₁ represents alkyl, alkenyl, alkynyl or aryl which contain up to 24 carbon atoms and which may contain up to 20 heteroatoms selected from nitrogen, oxygen, phosphorus and sulfur, or aralkyl which may contain one or more heteroatoms;
R₂, R₃, R₅ and R₇ independently represent hydrogen, amino, amido, acyl, alkoxyl, hydroxyl, -COON, halide, nitro, -CN, -SO₃H, 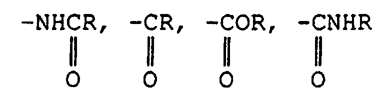 or -SCN; wherein R represents alkyl, alkenyl, alkynyl or aryl which contain up to 24 carbon atoms and which may contain up to 20 heteroatoms selected from nitrogen, oxygen, phosphorus and sulfur, or aralkyl which may contain one or more heteroatoms;
R₄ and R₈ independently represent alkyl, alkenyl, alkynyl, aralkyl or alkoxyl, which contain up to 8 carbon atoms;
R₆ represents Q-R-Nu or 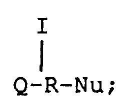 wherein Q represents -O-, -S-, -NH-, 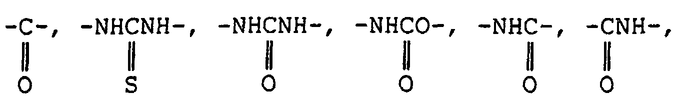 diazo or 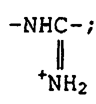 R is as defined above; I represents -SO₃H, -OSO₃H, -PO(OH)₂, -OPO(OH)₂ or -COOH; Nu represents the nucleophilic group amino; and X represents an anion; provided that, when Q represents -C(O)- and R represents -CH₃, I represents other than -SO₃H, and, when Q represents -C(O)- and R represents -C₂H₅, I represents other than -PO(OH)₂.

2. An acridinium ester as claimed in claim 1 wherein R₁ represents alkyl, alkenyl, alkynyl or aryl, which contain up to 24 carbon atoms and which may contain up to 10 heteroatoms selected from nitrogen, oxygen, phosphorus and sulfur;
R₂, R₃, R₅ and R₇ independently represent hydrogen, amino, -COOH, -CN, hydroxyl, C₁-C₄ alkoxyl, nitro, halide, -SO₃H or -SCN;
R₄ and R₈ independently represent alkyl, alkenyl, alkynyl or alkoxyl, which contain up to 8 carbon atoms;
X represents halide, CH₃SO₄⁻, FSO₃⁻, CF₃SO₃⁻, C₄F₉SO₃⁻, or 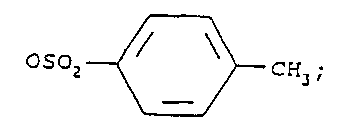 R contains up to 24 carbon atoms and may contain up to 10 heteroatoms selected from nitrogen, oxygen, phosphorus and sulfur; and
Nu represents amino.

3. An acridinium ester as claimed in claim 1 or claim 2 wherein R₁ represents C₁-C₁₀ alkyl; R₂, R₃, R₅ and R₇ independently represent hydrogen, C₁-C₄ alkoxyl, -CN, -SO₃H, nitro or amino; R₄ and R₈ independently represent C₁-C₄ alkyl; and X represents halide.

4. An acridinium ester as claimed in any of claims 1 to 3 wherein R₁, R₄ and R₈ represent methyl; R₂, R₃, R₅ and R₇ represent hydrogen; X represents bromide; and R₆ represents -CONH-CH₂CH₂-NH₂ or -CONH-CH₂CH₂NHCOCH-NH₂
CH₂S- (CH₂)₃SO₃H.

5. A luminescent conjugate **characterised in that** it comprises
an acridinium ester as claimed in any of claims 1 to 4 covalently-bound to a biologically-active molecule.

6. A luminescent conjugate as claimed in claim 5 wherein the biologically-active molecule is a protein, an antigen, a hapten, a nucleic acid, or a molecule comprising nucleic acids; preferably vitamin B₁₂ or a derivative thereof; or folate or a derivative thereof; or 17-beta-estradiol or a derivative thereof; or cortisol or a derivative thereof; or thromboxane B₂ or a prostaglandin analog.

7. A process for the production of a conjugate of folate or a derivative thereof and an acridinium ester as claimed in any of claims 1 to 4 **characterised in that** it comprises:
(a) incubating the folate (derivative) with an amino-reactive compound to couple an acyl or alkyloxy carbonyl group to the C-2 amino group of the folate (derivative) so as to form a protected folate intermediate;
(b) activating at least one carboxylic group of the protected folate intermediate;
(c) incubating the activated protected folate intermediate with the acridinium ester so as to form an acridinium ester-protected folate intermediate conjugate; and
(d) incubating the acridinium ester-protected folate intermediate conjugate in an acidic medium to remove the amino-protecting group from the C-2 amino group so as to form an acridinium ester-folate conjugate.

8. A process for the production of a conjugate of vitamin B₁₂ or a derivative thereof and an acridinium ester as claimed in any of claims 1 to 4 **characterised in that** it comprises:
(a) incubating the vitamin B₁₂ (derivative) in an acidic medium to deaminate the primary propanamide side chains of the Corrin ring of the vitamin B₁₂ (derivative) so as to produce a mixture of carboxylated vitamin B₁₂;
(b) subjecting the mixture of carboxylated vitamin B₁₂ to HPLC to separate the tricarboxylic, the dicarboxylic, and the three monocarboxylic vitamin B₁₂ forms;
(c) isolating one of the monocarboxylic vitamin B₁₂ forms;
(d) activating the carboxylic group of the monocarboxylated vitamin B₁₂; and
(e) incubating the activated monocarboxylic vitamin B₁₂ with the acridinium ester so as to produce an acridinium ester-vitamin B₁₂ conjugate.

9. A process for the production of a conjugate of 17-beta-estradiol or a derivative thereof or cortisol or a derivative thereof and an acridinium ester as claimed in any of claims 1 to 4 **characterised in that** it comprises:
(a) activating a functional group attached to the 17-beta-estradiol (derivative) or cortisol (derivative) so as to form an activated estradiol or cortisol intermediate; and
(b) incubating the activated estradiol or cortisol intermediate with the acridinium ester so as to form an acridinium ester-17-beta-estradiol or -cortisol conjugate.

10. A process for the production of a conjugate of thromboxane B₂ or a prostaglandin and an acridinium ester as claimed in any of claims 1 to 4 **characterised in that** it comprises:
(a) activating the terminal carboxylic group of the thromboxane B₂ or prostaglandin analog so as to form an activated intermediate of the thromboxane B₂ or the prostaglandin analog; and
(b) incubating the activated intermediate with the acridinium ester so as to form an acridinium ester-thromboxane B₂ or acridinium ester-prostaglandin analog conjugate.

11. The use of an acridinium ester as claimed in any of claims 1 to 4 or a conjugate as claimed in claim 5 or claim 6 or produced by a process as claimed in any of claims 7 to 10 as a chemiluminescent label in an assay system.

12. A method for the determination of vitamin B₁₂ or folate **characterised in that** it comprises:
(a) incubating a sample fluid under conditions which will release vitamin B₁₂ or folate in the sample from any endogenous vitamin B₁₂ - or folate - binding proteins so as to produce a released sample fluid;
(b) incubating the released sample fluid with (i) a composite comprising a vitamin B₁₂ - or folate - binding protein immobilized on a solid support and (ii) a conjugate as claimed in claim 6 or produced by a process as claimed in claim 8 or claim 7 so as to form a complexed composite;
(c) separating the complexed composite from any unbound conjugate;
(d) measuring the amount of acridinium ester label associated with the complexed composite; and
(e) determining the amount of vitamin B₁₂ or folate in the sample fluid from that measurement.

13. A method for the determination of vitamin B₁₂ or folate **characterised in that** it comprises:
(a) incubating a sample fluid with (i) a base, preferably sodium hydroxide, and (ii) a sulfhydryl compound, preferably dithiothreitol, so as to produce a released sample fluid;
(b) incubating the released sample fluid with (i) a composite comprising a vitamin B₁₂ - or folate - binding protein immobilized on a solid support and (ii) a conjugate as claimed in claim 6 or produced by a process as claimed in claim 8 or claim 7 so as to form a complexed composite;
(c) separating the complexed composite from any unbound conjugate;
(d) incubating the complexed composite with a thiol-reactive compound, preferably ethyl maleimide;
(e) measuring the amount of acridinium ester label associated with the complexed composite; and
(f) determining the amount of vitamin B₁₂ or folate in the sample fluid from that measurement.

14. A method for the determination of cortisol **characterised in that** it comprises:
(a) incubating a sample fluid with (i) a composite comprising an anti-cortisol antibody immobilized on a solid support and (ii) a conjugate as claimed in claim 6 or produced by a process as claimed in claim 9 so as to form a complexed composite;
(b) separating the complexed composite from any unbound conjugate;
(c) measuring the amount of acridinium ester label associated with the complexed composite; and
(d) determining the amount of cortisol in the sample fluid from that measurement.

15. A method for the determination of 17-beta-estradiol or thromboxane B₂ **characterised in that** it comprises:
(a) incubating a sample fluid with (i) an anti-17-beta-estradiol antibody or an anti-thromboxane B₂ antibody and (ii) a conjugate as claimed in claim 6 or produced by a process as claimed in claim 9 or claim 10 so as to form a reaction mixture;
(b) incubating the reaction mixture with a composite comprising an antibody capable of binding to anti-17-beta-estradiol or anti-thromboxane B₂ antibody, which antibody is immobilized on a solid support, to form a complexed composite;
(c) separating the complexed composite from the supernatant;
(d) measuring the amount of acridinium ester label associated with the complexed composite; and
(e) determining the amount of 17-beta-estradiol or thromboxane B₂ in the sample fluid from that measurement.

## Patentansprüche

1. Acridiniumester, **dadurch gekennzeichnet, dass** er der nachfolgenden allgemeinen Formel entspricht: 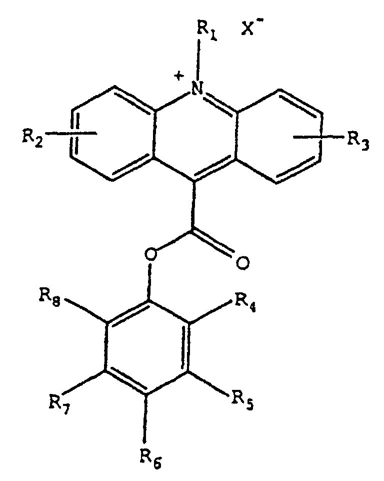 wobei R₁ Alkyl, Alkenyl, Alkinyl oder Aryl, die bis zu 24 Kohlenstoffatome enthalten und die bis zu 20 Heteroatome enthalten können, ausgewählt aus Stickstoff, Sauerstoff, Phosphor und Schwefel, oder Aralkyl, das ein oder mehrere Heteroatome enthalten kann, repräsentiert;
wobei R₂, R₃, R₅ und R₇ unabhängig Wasserstoff, Amino, Amido, Acyl, Alkoxyl, Hydroxyl, -COOH, Halogenid, Nitro, -CN, -SO₃H, 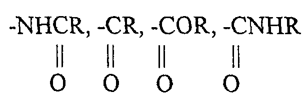 oder -SCN repräsentiert; wobei R Alkyl, Alkenyl, Alkinyl oder Aryl, die bis zu 24 Kohlenstoffatome enthalten und die bis zu 20 Heteroatome enthalten können, ausgewählt aus Stickstoff, Sauerstoff, Phosphor und Schwefel, oder Aralkyl, das ein oder mehrere Heteroatome enthalten kann, repräsentiert;
wobei R₄ und R₈ unabhängig Alkyl, Alkenyl, Alkinyl, Aralkyl oder Alkoxyl repräsentieren, die bis zu 8 Kohlenstoffatome enthalten;
wobei R₆ Q-R-Nu oder 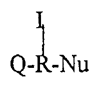 repräsentiert; wobei Q -O-, -S-, -NH-, 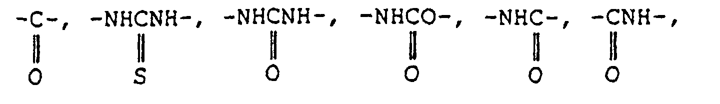 Diazo oder 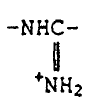 repräsentiert; wobei R wie oben definiert ist; I -SO₃H, -OSO₃H, - PO(OH)₂, -OPO(OH)₂ oder -COOH repräsentiert; Nu die nukleophile Gruppe Amino repräsentiert; und X ein Anion repräsentiert, vorausgesetzt dass, wenn Q -C(O)- repräsentiert und R -CH₃ repräsentiert, I etwas anderes als -SO₃H repräsentiert, und, wenn Q -C(O)-repräsentiert und R -C₂H₅ repräsentiert, I etwas anderes als -PO(OH)₂ repräsentiert.

2. Acridiniumester nach Anspruch 1, wobei R₁ als Alkyl, Alkenyl, Alkinyl oder Aryl repräsentiert, die bis zu 24 Kohlenstoffatome enthalten und die bis zu 10 Heteroatome enthalten können, ausgewählt aus Stickstoff, Sauerstoff, Phosphor und Schwefel;
wobei R₂, R₃, R₅ und R₇ unabhängig Wasserstoff, Amino, -COOH, -CN, Hydroxyl, C₁-C₄ Alkoxyl, Nitro, Halogenid, -SO₃H oder -SCN repräsentiert;
R₄ und R₈ unabhängig Alkyl, Alkenyl, Alkinyl oder Alkoxyl repräsentieren, die bis zu 8 Kohlenstoffatome enthalten;
X Halogen, CH₃SO₄₋, FSO₃₋, CF₃SO₃₋, C₄F₉SO₃₋, oder 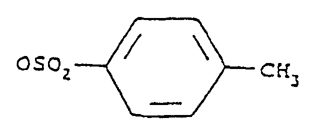 repräsentiert, wobei R bis zu 24 Kohlenstoffatome enthält und bis zu 10 Heteroatome enthalten kann, die aus Stickstoff, Sauerstoff, Phosphor und Schwefel ausgewählt sind; und Nu Amino repräsentiert

3. Acridiniumester nach Anspruch 1 oder Anspruch 2, bei dem R₁ C₁-C₁₀ Alkyl; R₂, R₃, R₅ und R₇ unabhängig Wasserstoff, C₁-C₄ Alkoxyl, -CN, SO₃H, Nitro oder Amino repräsentieren; R₄ und R₈ unabhängig C₁-C₄ Alkyl repräsentieren; und X Halogenid repräsentiert.

4. Acridiniumester nach einem der Ansprüche 1-3, wobei R₁, R₄ und R₈ Methyl repräsentieren, R₂, R₃, R₅ und R₇ Wasserstoff repräsentieren; X Bromid repräsentiert; und R₆ -CONH-CH₂CH₂-NH₂ oder 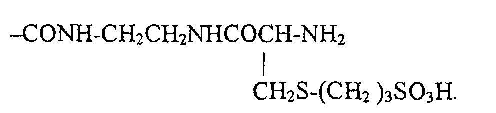 repräsentiert.

5. Lumineszierendes Konjugat, **dadurch gekennzeichnet, dass** es einen Acridiniumester nach einem der Ansprüche 1-4 umfasst, der kovalent an ein biologisch aktives Molekül gebunden ist.

6. Lumineszierendes Konjugat nach Anspruch 5, wobei das biologisch aktive Molekül ein Protein, ein Antigen, ein Hapten, eine Nukleinsäure oder ein Molekül, das Nukleinsäuren umfasst; vorzugsweise Vitamin B₁₂ oder ein Derivat hiervon; oder Folat oder ein Derivat hiervon; oder 17-beta-Estradiol oder ein Derivat hiervon; oder Koitison oder ein Derivat hiervon; oder Thromboxan B₂ oder ein Prostaglandin-Analogon, ist.

7. Verfahren zur Herstellung eines Konjugats aus Folat oder eines Derivats hiervon und aus einem Acridiniumester nach einem der Ansprüche 1-4, **dadurch gekennzeichnet, dass** es folgendes umfasst:
(a) Inkubieren des Folats (Derivats) mit einer Amino-reaktiven Verbindung, um eine Acyl-oder Alkyloxycarbonyl-Gruppe an die C-2 Aminogruppe des Folats (Derivats) zu binden, so dass ein geschütztes Folat-Zwischenprodukt gebildet wird;
(b) Aktivieren zumindest einer Carboxylgruppe des geschützten Folat-Zwischenprodukts;
(c) Inkubieren des aktivierten geschützten Folat- Zwischenprodukts mit dem Acridiniumester, so dass ein Konjugat aus Acridiniumester-geschütztem Folat-Zwischenprodukt gebildet wird; und
(d) Inkubieren des Konjugats aus Acridiniumester-geschütztem Folat-Zwischenprodukt in einem sauren Medium, um die Aminoschutzgruppe von der C-2 Aminogruppe zu entfernen, so dass ein Acridiniumester-Folat-Konjugat gebildet wird.

8. Verfahren zur Herstellung eines Konjugats aus Vitamin B₁₂ oder eines Derivats hiervon und aus einem Acridiniumester nach einem der Ansprüche 1-4, **dadurch gekennzeichnet, dass** es folgendes umfasst:
(a) Inkubieren des Vitamins B₁₂ (Derivats) in einem sauren Medium, um die primären Propanamid-Seitenketten des Corrin-Rings des Vitamin B₁₂ (Derivats) zu desaminieren, so dass ein Gemisch aus carboxyliertem Vitamin B₁₂ erzeugt wird;
(b) Unterwerfen des Gemisches aus carboxyliertem Vitamin B₁₂ einer HPLC, um die Tricarboxyl, die Dicarboxyl und die drei Monocarboxyl-Vitamin B₁₂ Formen zu trennen;
(c) Isolieren einer der Monocarboxyl-Vitamin B₁₂ Formen;
(d) Aktivieren der Carboxylgruppe des monocarboxylierten Vitamin B₁₂; und
(e) Inkubieren des aktivierten Monocarboxyl-Vitamin B₁₂ mit dem Acridiniumester, so dass ein Konjugat aus Acridiniumester-Vitamin B₁₂ hergestellt wird.

9. Verfahren zur Herstellung eines Konjugats aus 17-beta-Estradiol oder eines Derivats hiervon oder von Kortison oder eines Derivates hiervon und aus einem Acridiniumester nach einem der Ansprüche 1-4, **dadurch gekennzeichnet, dass** es folgendes umfasst:
(a) Aktivieren einer funktionellen Gruppe, die an dem 17-beta-Estradiol (Derivat) oder Kortison (Derivat) gebunden ist, so dass ein aktiviertes Estradiol oder Kortison- Zwischenprodukt gebildet wird; und
(b) Inkubieren des aktivierten Estradiols oder Kortison-Zwischenprodukts mit dem Acridiniumester, so dass ein Konjugat aus Acridiniumester - 17-beta-Estradiol oder -Koritison gebildet wird.

10. Verfahren zur Herstellung eines Konjugats aus Thromboxan B₂ oder einem Prostaglandin und einem Acridiniumester nach einem der Ansprüche 1-4, **dadurch gekennzeichnet, dass** es folgendes umfasst:
(a) Aktivieren der terminalen Carboxylgruppe des Thromboxans B₂ oder des Prostaglandin-Analogons, so dass ein aktiviertes Zwischenprodukt des Thromboxan B₂ oder des Prostaglandin-Analogons gebildet wird, und
(b) Inkubieren des aktivierten Zwischenproduktes mit dem Acridiniumester, so dass ein Konjugat aus Acridiniumester - Thromboxan B₂ oder Acridiniumester - Prostaglandin-Analogon gebildet wird.

11. Verwendung eines Acridiniumesters nach einem der Ansprüche 1-4 oder eines Konjugats nach Anspruch 5 oder Anspruch 6 oder hergestellt durch ein Verfahren nach einem der Ansprüche 7 bis 10, als chemilumineszierende Markierung in einem Assay-System.

12. Verfahren zur Bestimmung von Vitamin B₁₂ oder Folat, **dadurch gekennzeichnet, dass** es folgendes umfasst:
(a) Inkubieren einer Probenflüssigkeit unter Bedingungen, die Vitamin B₁₂ oder Folat in der Probe aus irgendwelchen endogenen Vitamin B₁₂- oder Folatbindenden Proteinen freisetzt, so dass eine freigesetzte Probenflüssigkeit erzeugt wird;
(b) Inkubieren der freigesetzten Probenflüssigkeit mit (i) einem Gemisch, das ein Vitamin B₁₂- oder Folat-Bindungsprotein, immobilisiert an einem festen Träger umfasst und (ii) mit einem Konjugat nach Anspruch 6 oder das durch ein Verfahren nach Anspruch 8 oder Anspruch 7 hergestellt wurde, so dass ein komplexiertes Gemisch gebildet wird;
(c) Abtrennen des komplexierten Gemisches von jedem ungebundenen Konjugat;
(d) Messen der Menge an Acridiniumester-Markierung, die mit dem komplexierten Gemisch verbunden ist; und
(e) Bestimmen der Menge an Vitamin B₁₂ oder Folat in der Probenflüssigkeit aus dieser Messung.

13. Verfahren zur Bestimmung von Vitamin B₁₂ oder Folat, **dadurch gekennzeichnet, dass** es folgendes umfasst:
(a) Inkubieren einer Probenflüssigkeit mit (i) einer Base, vorzugsweise Natriumhydroxid, und (ii) einer Sulfhydrylverbindung, vorzugsweise Dithiothreitol, so dass eine freigesetzte Probenflüssigkeit erzeugt wird;
(b) Inkubieren der freigesetzten Probenflüssigkeit mit (i) einem Gemisch, dass ein Vitamin B₁₂- oder Folat-Bindungsprotein, immobilisiert an einem festen Träger, und (ii) mit einem Konjugat nach Anspruch 6 oder wie durch ein Verfahren nach Anspruch 8 oder Anspruch 7 produziert, so dass ein komplexiertes Gemisch gebildet wird;
(c) Trennen des komplexierten Gemisches von jedem ungebundenen Konjugat;
(d) Inkubieren des komplexierten Gemisches mit einer Thiol-reaktiven Verbindung, vorzugsweise Ethylmaleimid;
(e) Messen der Menge an Acridiniumester-Markierung, die mit dem komplexierten Gemisch verbunden ist; und
(f) Bestimmen der Menge an Vitamin B₁₂ oder Folat in der Probenflüssigkeit aus dieser Messung.

14. Verfahren zur Bestimmung von Kortison, **dadurch gekennzeichnet, dass** es folgendes umfasst:
(a) Inkubieren einer Probenflüssigkeit mit (i) einem Gemisch, das einen Anti-Kortison Antikörper immobilisiert an einen festen Träger umfasst, und (ii) mit einem Konjugat nach Anspruch 6 oder das durch ein Verfahren nach Anspruch 10 produziert wird, so dass ein komplexiertes Gemisch gebildet wird;
(b) Abtrennen des komplexierten Gemisches von jedem ungebundenen Konjugat;
(c) Messen der Menge an Acridiniumester-Markierung, die mit dem komplexierten Gemisch verbunden ist; und
(d) Bestimmen der Menge an Kortison in der Probenflüssigkeit aus dieser Messung.

15. Verfahren zur Bestimmung von 17-beta-Estradiol oder Thomboxan B₂, **dadurch gekennzeichnet, dass** es folgendes umfasst:
(a) Inkubieren einer Probenflüssigkeit mit (i) einem Anti-17-beta-Estradiol-Antikörper oder einem anti-Thromboxan B₂-Antikörper und (ii) mit einem Konjugat nach Anspruch 6 oder einem, dass durch ein Verfahren nach Anspruch 9 oder Anspruch 11 hergestellt wurde, um ein Reaktionsgemisch zu bilden;
(b) Inkubieren des Reaktionsgemisches mit einem Gemisch, das einen Antikörper umfasst, der zur Bindung an einen anti-17-beta-Estradiol- oder Anti-Thromboxan B₂-Antikörper in der Lage ist, wobei der Antikörper an einem festen Träger immobilisiert ist, zur Bildung eines komplexierten Gemisches;
(c) Abtrennen des komplexierten Gemisches aus dem Überstand;
(d) Messen der mit dem komplexiertem Gemisch verbundenen Menge an Acridiniumester-Markierung; und
(e) Bestimmen der Menge von 17-beta-Estradiol oder Thromboxan B₂ in der Probenflüssigkeit aus dieser Messung.

## Revendications

1. Un ester d'acridinium **caractérisé en ce qu'**il correspond à la formule générale suivante : 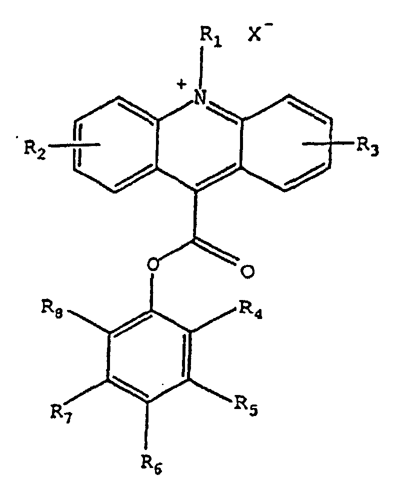 dans laquelle
R₁ représente un groupe alkyle, alcényle, alcynyle ou aryle qui contient jusqu'à 24 atomes de carbone et qui peut contenir jusqu'à 20 hétéroatomes choisis parmi l'azote, l'oxygène, le phosphore et le soufre, ou un groupe aralkyle qui peut contenir un ou plusieurs hétéroatomes ;
R₂, R₃, R₅ et R₇ représentent indépendamment l'hydrogène, un groupe amino, amido, acyle, alcoxy, hydroxyle, -COOH, halogénure, nitro, -CN, -SO₃H, 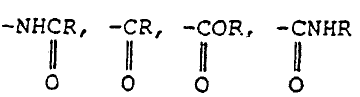 ou -SCN ; où R représente un groupe alkyle, alcényle, alcynyle ou aryle qui contient jusqu'à 24 atomes de carbone et qui peut contenir jusqu'à 20 hétéroatomes choisis parmi l'azote, l'oxygène, le phosphore et le soufre, ou un groupe aralkyle qui peut contenir un ou plusieurs hétéroatomes ;
R₄ et R₈ représentent indépendamment un groupe alkyle, alcényle, alcynyle, aralkyle ou alcoxy qui contient jusqu'à 8 atomes de carbone ;
R₆ représente Q-R-Nu ou 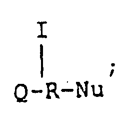 où Q représente -O-, -S-, -NH- 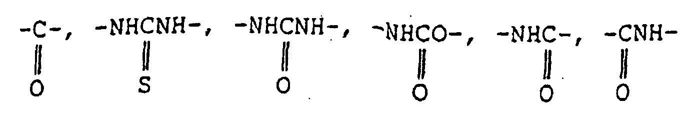 diazo ou 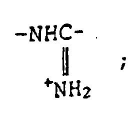 R est tel que défini ci-dessus ; I représente -SO₃H, -OSO₃H, -PO(OH)₂, -OPO(OH)₂ ou -COOH ; Nu représente le groupe nucléophile amino ; et X représente un anion ; à condition que si Q représente -C(O)- et R représente -CH₃, alors I représente autre chose que -SO₃H, et que si Q représente -C(O)- et R représente -C₂H₅, alors I représente autre chose que -PO(OH)₂.

2. Un ester d'acridinium selon la revendication 1, dans lequel R₁ représente un groupe alkyle, alcényle, alcynyle ou aryle, qui contient jusqu'à 24 atomes de carbone et qui peut contenir jusqu'à 10 hétéroatomes choisis parmi l'azote, l'oxygène, le phosphore et le soufre ;
R₂, R₃, R₅ et R₇ représentent indépendamment l'hydrogène, un groupe amino, -COOH, -CN, hydroxyle, alcoxy en C₁-C₄, nitro, halogénure, -SO₃H ou -SCN ;
R₄ et R₈ représentent indépendamment un groupe alkyle, alcényle, alcynyle ou alcoxy, pouvant contenir jusqu'à 8 atomes de carbone ;
X représente un halogénure, CH₃SO₄⁻, FSO₃⁻, CF₃SO₃⁻, C₄F₉SO₃⁻ ou 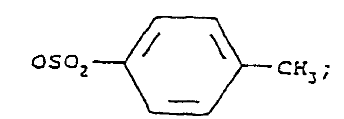 R contient jusqu'à 24 atomes de carbone et peut contenir jusqu'à 10 hétéroatomes choisis parmi l'azote, l'oxygène, le phosphore et le soufre ; et Nu représente un groupe amino.

3. Un ester d'acridinium selon la revendication 1 ou la revendication 2, dans lequel R₁ représente un groupe alkyle en C₁-C₁₀ ; R₂, R₃, R₅ et R₇ représentent indépendamment l'hydrogène, un groupe alcoxy en C₁-C₄, -CN, -SO₃H, nitro ou amino ; R₄ et R₈ représentent indépendamment un groupe alkyle en C₁-C₄ ; et X représente un halogénure.

4. Un ester d'acridinium selon l'une quelconque des revendications 1 à 3, dans lequel R₁, R₄ et R₈ représentent un groupe méthyle ; R₂, R₃, R₅ et R₇ représentent l'hydrogène ; X représente le bromure ; et R₆ représente -CONH-CH₂CH₂-NH₂ ou

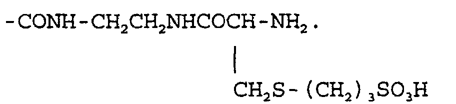

5. Un conjugué luminescent **caractérisé en ce qu'**il comprend un ester d'acridinium tel que revendiqué dans l'une quelconque des revendications 1 à 4, lié par covalence à une molécule biologiquement active.

6. Un conjugué luminescent selon la revendication 5, dans lequel la molécule biologiquement active est une protéine, un antigène, un haptène, un acide nucléique ou une molécule comprenant des acides nucléiques ; de préférence la vitamine B₁₂ ou un dérivé de celle-ci ; ou un folate ou un dérivé de celui-ci ; ou le 17-bêta-oestradiol ou un dérivé de celui-ci ; ou le cortisol ou un dérivé de celui-ci ; ou le thromboxane B₂ ou un analogue de prostaglandine.

7. Un procédé pour la production d'un conjugué de folate ou dérivé de celui-ci et d'un ester d'acridinium tel que revendiqué dans l'une quelconque des revendications 1 à 4, **caractérisé en ce qu'**il comprend les opérations consistant à :
(a) faire incuber le folate (dérivé) avec un composé réactif avec la fonction amine pour coupler un groupe acyle ou alkyloxycarbonyle au groupe amino en C-2 du folate (dérivé) de manière à former un folate protégé intermédiaire ;
(b) activer au moins un groupe carboxylique du folate protégé intermédiaire ;
(c) faire incuber avec l'ester d'acridinium le folate protégé intermédiaire activé, de manière à former un conjugué ester d'acridinium-folate protégé intermédiaire ; et
(d) faire incuber le conjugué ester d'acridinium-folate protégé intermédiaire dans un milieu acide pour éliminer le groupe protecteur d'amine du groupe amino en C-2 de manière à former un conjugué ester d'acridinium-folate.

8. Un procédé pour la production d'un conjugué de vitamine B₁₂ ou dérivé de celle-ci et d'un ester d'acridinium tel que revendiqué dans l'une quelconque des revendications 1 à 4, **caractérisé en ce qu'**il comprend les opérations consistant à :
(a) faire incuber la vitamine B₁₂ (dérivé) dans un milieu acide pour désaminer les chaînes latérales de propanamide primaire du cycle de corrine de la vitamine B₁₂ (dérivé) de manière à produire un mélange de vitamines B₁₂ carboxylées ;
(b) soumettre le mélange de vitamines B₁₂ carboxylées à une CLHP pour séparer la forme tricarboxylique, la forme dicarboxylique et les trois formes monocarboxyliques de vitamine B₁₂ ;
(c) isoler l'une des formes monocarboxyliques de vitamine B₁₂ ;
(d) activer le groupe carboxylique de la vitamine B₁₂ monocarboxylée ; et
(e) faire incuber avec l'ester d'acridinium la vitamine B₁₂ monocarboxylée activée de manière à produire un conjugué ester d'acridinium-vitamine B₁₂.

9. Un procédé pour la production d'un conjugué de 17-bêta-oestradiol ou dérivé de celui-ci ou de cortisol ou dérivé de celui-ci et d'un ester d'acridinium tel que revendiqué dans l'une quelconque des revendications 1 à 4, **caractérisé en ce qu'**il comprend les opérations consistant à :
(a) activer un groupe fonctionnel fixé au 17-bêta-oestradiol (dérivé) ou cortisol (dérivé) de manière à former un oestradiol ou cortisol activé intermédiaire ; et
(b) faire incuber l'oestradiol ou le cortisol activé intermédiaire avec l'ester d'acridinium de manière à former un conjugué ester d'acridinium-17-bêta-oestradiol ou -cortisol.

10. Un procédé pour la production d'un conjugué de thromboxane B₂ ou de prostaglandine et d'un ester d'acridinium tel que revendiqué dans l'une quelconque des revendications 1 à 4, **caractérisé en ce qu'**il comprend les opérations consistant à :
(a) activer le groupe carboxylique terminal du thromboxane B₂ ou de l'analogue de prostaglandine de manière à former un intermédiaire activé du thromboxane B₂ ou de l'analogue de prostaglandine ; et
(b) faire incuber l'intermédiaire activé avec l'ester d'acridinium de manière à former un conjugué ester d'acridinium-thromboxane B₂ ou ester d'acridinium-analogue de prostaglandine.

11. L'utilisation d'un ester d'acridinium tel que revendiqué dans l'une quelconque des revendications 1 à 4 ou d'un conjugué tel que revendiqué dans la revendication 5 ou la revendication 6 ou produit par un procédé tel que revendiqué dans l'une quelconque des revendications 7 à 10, comme marqueur chimioluminescent dans un système de dosage.

12. Une méthode pour la détermination de vitamine B₁₂ ou de folate, **caractérisée en ce qu'**elle comprend les opérations consistant à :
(a) faire incuber un échantillon de fluide dans des conditions qui libèrent la vitamine B₁₂ ou le folate contenu dans l'échantillon de toutes protéines endogènes liant la vitamine B₁₂ ou le folate de manière à produire un échantillon de fluide libéré ;
(b) faire incuber l'échantillon de fluide libéré avec (i) un composite comprenant une protéine liant la vitamine B₁₂ ou le folate immobilisée sur un support solide et (ii) un conjugué tel que revendiqué dans la revendication 6 ou produit par un procédé tel que revendiqué dans la revendication 8 ou la revendication 7 de manière à former un composite complexé ;
(c) séparer le composite complexé de tout conjugué non lié ;
(d) mesurer la quantité de marqueur ester d'acridinium associé au composite complexé ; et
(e) déterminer la quantité de vitamine B₁₂ ou de folate dans l'échantillon de fluide d'après cette mesure.

13. Une méthode pour la détermination de vitamine B₁₂ ou de folate, **caractérisée en ce qu'**elle comprend les opérations consistant à :
(a) faire incuber un échantillon de fluide avec (i) une base, de préférence l'hydroxyde de sodium, et (ii) un composé sulfhydrylé, de préférence le dithiothréitol, de manière à produire un échantillon de fluide libéré ;
(b) faire incuber l'échantillon de fluide libéré avec (i) un composite comprenant une protéine liant la vitamine B₁₂ ou le folate immobilisée sur un support solide et (ii) un conjugué tel que revendiqué dans la revendication 6 ou produit par un procédé tel que revendiqué dans la revendication 8 ou la revendication 7, de manière à former un composite complexé ;
(c) séparer le composite complexé de tout conjugué non lié ;
(d) faire incuber le composite complexé avec un composé réactif avec la fonction thiol, de préférence l'éthylmaléimide ;
(e) mesurer la quantité de marqueur ester d'acridinium associé au composite complexé ; et
(f) déterminer la quantité de vitamine B₁₂ ou de folate dans l'échantillon de fluide d'après cette mesure.

14. Une méthode pour la détermination du cortisol, **caractérisée en ce qu'**elle comprend les opérations consistant à :
(a) faire incuber un échantillon de fluide avec (i) un composite comprenant un anticorps anti-cortisol immobilisé sur un support solide et (ii) un conjugué tel que revendiqué dans la revendication 6 ou produit par un procédé tel que revendiqué dans la revendication 10 de manière à former un composite complexé ;
(b) séparer le composite complexé de tout conjugué non lié ;
(c) mesurer la quantité de marqueur ester d'acridinium associé au composite complexé ; et
(d) déterminer la quantité de cortisol dans l'échantillon de fluide d'après cette mesure.

15. Une méthode pour la détermination du 17-bêta-oestradiol ou du thromboxane B₂, **caractérisée en ce qu'**elle comprend les opérations consistant à :
(a) faire incuber un échantillon de fluide avec (i) un anticorps anti-17-bêta-oestradiol ou un anticorps anti-thromboxane B₂ et (ii) un conjugué tel que revendiqué dans la revendication 6 ou produit par un procédé tel que revendiqué dans la revendication 9 ou la revendication 11 de manière à former un mélange réactionnel ;
(b) faire incuber le mélange réactionnel avec un composite comprenant un anticorps capable de se lier à l'anticorps anti-17-bêta-oestradiol ou anti-thromboxane B₂, lequel anticorps est immobilisé sur un support solide, pour former un composite complexé ;
(c) séparer le composite complexé du surnageant ;
(d) mesurer la quantité de marqueur ester d'acridinium associé au composite complexé ; et
(e) déterminer la quantité de 17-bêta-oestradiol ou de thromboxane B₂ dans l'échantillon de fluide d'après cette mesure.
